# EUROPÄISCHE PATENTSCHRIFT

(11) **EP 0 999 806 B1**
(45) Veröffentlichungstag und Bekanntmachung des Hinweises auf die Patenterteilung: **10.04.2002**
(21) Anmeldenummer: 98947360.8
(22) Anmeldetag: 23.07.1998
(51) Int. Cl.: A61F 2/06

(54) **INTRALUMINALE IMPLANTATIONSVORRICHTUNG**
INTRALUMINAL IMPLANTATION DEVICE
DISPOSITIF D'IMPLANTATION INTRALUMINAL

(30) Priorität: 24.07.1997 DE 19731834
(43) Veröffentlichungstag der Anmeldung: 17.05.2000
(73) Patentinhaber: Strecker, Ernst Peter, Dr.-med.Prof., D-76228 Karlsruhe (DE)
(72) Erfinder: Strecker, Ernst Peter, Dr.-med.Prof., D-76228 Karlsruhe (DE)
(74) Vertreter: Geitz, Holger
(86) Internationale Anmeldenummer: DE9802136
(87) Internationale Veröffentlichungsnummer: WO9904724

(56) Entgegenhaltungen:
- EP-A- 0 380 666
- EP-A- 0 466 518
- WO-A-90/15582
- US-A- 4 872 874
- US-A- 5 254 127

## Beschreibung

Die Erfindung betrifft eine lmplantationsvorrichtung zur Behandlung von im Bereich der Innenwandung von Hohlorganen verletztem oder erkranktem Gewebe, insbesondere Implantationsvorrichtung zur Behandlung einer Dissektion in Körpergefäßen,mit einem Katheter und wenigstens einem mit diesem Katheters verbundenen Befestigungsmittel wobei der Katheter einen langgestreckten, an zumindest seinem bei bestimmungsgemäßen Gebrauch vorderen Ende offenen Hohlkörper aufweist, in dem jedes Befestigungsmittel beim Einführen der Implantationsvorrichtung in das Körpergefäß axialbeweglich aufgenommen ist.

Eine solche Implantationsvorrichtung, wenn auch nicht zur Behandlung von Dissektionen in Körpergefäßen, ist aus der EP - A - 466 518 vorbekannt.

Es handelt sich dabei um eine Implantationsvorrichtung für ein künstliches Blutgefäß, das mittels eines Katheters lagerichtig implantiert und am Impantationsort aufgedehnt wird. Hierzu ist das künstliche Blutgefäß während der Implatation in einer einschnürenden Umhüllung aufgenommen, die nach der Implantation entfernt wird. Das künstliche Blutgefäß ist mit Federelementen, die ihrerseits Widerhaken aufweisen versehen, die sich am Implantationsort verhaken und so das künstliche Blutgefäß federnd in seiner gewünschten Lage halten und z.T. wunschgemäß aufdehnen. Dadurch daß die federnden Befestigunsmittel mit dem künstlichen Blutgefäß und insoweit allenfalls indirekt mit dem Katheter verbunden sind ist eine genaue Positionierung der Befestigungsmittel, wie sie beispielsweise zur Behandlung von Dissektionen aber auch im Zusammenhang mit anderen Anwendungen wünschenswert ist, nicht möglich. Die Befestigungsmittel sind beim Gegenstand der EP - A - 466 518 vielmehr fest mit dem künstlichen Blutgefäß verbunden. Eine Implantation der Befestigungsmittel als solcher ist demnach mit dieser vorbekannten Implantationsvorrichtung nicht möglich.

Aus der US-PS 4872 874 sind bereits ein Verfahren und eine Einrichtung zum Befestigen eines Grafts im Lumen eine Körpergefäßes vorbekannt. Die vorbekannte Einrichtung umfaßt einen Katheter mit einem aufweitbaren Ballonabschnitt an seinem distalen Ende, der Ballonabschnitt ist mit einer U-förmigen Ringnut versehen, in der über den Umfang verteilt als Befestigungsmittel Heftklammern mit über einen Steg miteinander verbundenen Schenkeln so aufnehmbar sind, daß die freien Enden der Schenkel nach außen weisen. Mittels dieser Einrichtung sind zuvor in ein Körpergefäß eingebrachte Grafts an Gefäßuandungen befestigbar, indem der im nicht aufgeweiteten Zustand in das Gefäß eingeführte, mit Befestigungsmitteln bestückte Ballonabschnitt mit der die Befestigungsmittel aufnehmenden Ringnut in der Nähe eines Graftendes positioniert und dann der Ballonabschnitt aufgeweitet wird. Dabei sollen die radial vorstehenden Schenkel der Heftklammern den Graft durchstoßen und in die Gefäßwand eindringen, um nach dem Kollabieren des Ballonabschnittes den Graft an der Gefäßwand zu halten.

Es hat sich indessen gezeigt, daß die Schenkel der als Befestigungsmittel dienenden Heftklammern sich beim Aufdehnen des Ballonabschnittes leicht schräg stellen und gar nicht oder nur unzulänglich in die Gefäßwand eindringen. Dies dürfte darauf zurückzuführen sein, daß die Tiefe der als Aufnahme für die Befestigungsmittel dienende Ringnut beim Aufdehnen des Ballonabschnittes fortschreitend kleiner wird und dadurch die Befestigungsmittel ihre Führung verlieren. Als Folge davon wird eine nur unzulängliche Anheftung des Grafts an die Gefäßwand erreicht und es können darüber hinaus Schädigungen der Gefäßwand auftreten.

Bei der vorbekannten Einrichtung weist der Ballonabschnitt des Katheters bereits im nicht aufgedehnten Zustand eine beträchtliche radiale Ausdehnung auf, so daß diese Implantationseinrichtung nur bei größeren Körpergefäßen einsetzbar ist.

Demgemäß besteht die der Erfindung zugrundeliegende Aufgabe in der Schaffung einer verbesserten Implantationsvorrichtung der vorgenannten Art und Zweckbestimmung, die. eine streng lokale Behandlung von verletztem oder erkrankten Gewebe der Innenwandung von Hohlorganen bei gleichzeitiger Reduzierung der Gewebebelastung ermöglicht.

Gelöst ist diese Aufgabe dadurch, daß bei der Implantationsvorrichtung nach dem Oberbegriff des Patentanspruchs 1 der Katheter einen langgestreckten Hohlkörper aufweist, der zumindest an seinem bei bestimmungsgemäßem Gebrauch vorderen Ende offen ist, und in dem das oder die Befestigungsmittel axialbeweglich aufgenommen ist/sind. Der Einsatz eines derartigen Katheters benötigt nur einen geringeren Einführquerschnitt und ist deshalb auch bei Gefäßen mit kleinem Lumen geeignet. Nach lagerichtiger Plazierung des Katheters wird das in diesem aufgenommene Befestigungsmittel aus dem Katheter ausgetrieben und in die Gefäßwand implantiert.

Um die Implantation des Befestigungsmittels zu erleichtern, ist der Hohlkörper des Katheters vorteilhafterweise an seinem vorderen Ende aufgebogen. Die Aufbiegung bewirkt auch eine durchaus wünschenswerte begrenzte Aufdehnung des Körpergefäßes während der Implantation.

Die Implantationsvorrichtung umfaßt somit einen Katheter, in dem während des Einführens in das Körpergefäß ein oder mehrere Befestigungsmittel aufgenommen ist/sind, die nach lagerichtigen Plazierung des Katheters im Bereich der erkrankten Gewebestelle, etwa an einem Dissekat, aus dem Katheter vorgetrieben und in die Gefäßwandung implantiert werden. Im Falle eines Dissekats erfolgt die Behandlung in einfacher Weise dadurch, daß das Dissekat mit Hilfe des Befestigungsmittels an die Gefäßwand angeheftet wird. Mit der erfindungsgemäßen Implantationsvorrichtung ist somit eine zielgenaue Behandlung des Dissekats gewährleistet. Das Gewebe wird lediglich im unmittelbaren Bereich der Befestigungsmittel beansprucht, mithin ist die Belastung für das Organgewebe auf ein Minimum reduziert.

Bei größeren Dissekaten empfiehlt sich der Einsatz mehrerer Befestigungsmittel, die voneinander beabstandet zu implantieren sind. Die Einsatzmöglichkeiten der erfindungsgemäßen Implantationsvorrichtung ,gehen weit über die Behandlung von Gefäßerkrankungen hinaus, und können sich beispielsweise auch auf die Behandlung von Netzhautablösungen erstrekken, indem die Netzhaut mit Hilfe von Befestigungsmitteln zuverlässig und dauerhaft mit der Aderhaut verbunden wird. Im Gegensatz zu gängigen koagulierenden Therapien, die lediglich eine prophylaktische Behandlung gefährdeter Areale gestatten, ist bei der Behandlung mit Hilfe der erfindungsgemäßen Implantationsvorrichtung auch das Anheften bereits abgelöster Areale der Netzhaut möglich.

Erweiterte Behandlungsmöglichkeiten ergeben sich dadurch, daß am Katheter Mittel zum Aufnehmen und Positionieren einer Schutzüberdeckung für das betroffene Gewebe angeordnet sind. Mit Hilfe der Schutzüberdeckung können auch großflächige Gewebebereiche behandelt werden, bei denen der Einsatz lediglich von Befestigungsmitteln nicht ausreicht oder nicht möglich ist. Insbesondere zur Behandlung von Aneurysmen empfiehlt sich die Abdeckung der Gefäßaussackung mit einer derartigen Schutzüberdeckung, die im Randbereich des Aneurysmas im gesunden Gewebe mit Befestigungsmitteln angeheftet wird. Im Gegensatz zur konventionellen Behandlung mit Stents wird das Gefäßlumen dabei nicht verengt und die Gefahr einer Stenose durch Bildung einer Neointima weitgehend vermindert.

Die Art der einzusetzenden Schutzüberdeckung hängt dabei von der jeweiligen Anwendung ab. Eine zweckmäßige Schutzüberdeckung ist etwa ein dem geschädigten Gewebeareal angepaßtes Textil- oder Gewebeteil. Entsprechend der Anwendung kann das Gewebe aus einem stabilen oder flexiblen Material bestehen. Für viele Anwendungen empfiehlt sich der Einsatz eines biologisch abbaubaren Materials. Insbesondere ist auch der Einsatz von körpereigenem Gewebe oder eines Transplantats möglich.

Die vorliegende Implantationsvorrichtung erlaubt auch das Anheften von Grafts an die Innenwandung eines Körpergefäßes. Damit kann auf eine stützende Stentstruktur völlig verzichtet werden. Im Gegensatz zu konventionellen Behandlungen mit Stents wird das Lumen des Körpergefäßes nicht nennenswert verkleinert.

Ein für viele Fälle ausreichendes Befestigungsmittel ist ein der Dicke der Wandung des Hohlorganes angepaßter Nagel, der mit seinem Schaft in die Gefäßwand eingebracht wird. Je nach Beanspruchung des betroffenen Gewebebereichs können jedoch auch Krampen mit zwei oder mehr Schenkeln zum Einsatz kommen, deren Schenkel U- oder V-förmig zueinander angeordnet sind. Auch kann das Befestigungsmittel zwei biegsame, an jeweils einem Ende miteinander verbundenen Schenkel aufweisen, die während der Implantation im Katheter parallel zueinander gehalten werden und erst während der Implantation V-förmig aufzweigen, wodurch einerseits der beim Einführen des Katheters in Anspruch genommene Querschnitt sehr gering gehalten wird und andererseits im Implantationszustand eine zuverlässige und feste Verbindung entsteht. Auch ist es möglich, das Befestigungsmittel mit Widerhaken auszustatten, wodurch abermals die Festigkeit der durch die Befestigungsmittel vermittelten Verbindung erhöht wird.

Auch bei den Befestigungsmitteln ist fallweise der Einsatz von biologisch abbaubarem Material von großem Vorteil. Bestehen die Befestigungsmittel aus einem elastischen Material, wie etwa Nitinol, wird das Einführen des die Befestigungsmittel enthaltenden Katheterabschnittes bei stark gekrümmten Körpergefäßen erleichtert und die Gefahr von Verletzungen reduziert.

Alternativ zu den aus festen Materialien bestehenden Befestigungsmitteln ist auch der Einsatz eines Materials möglich, das bei der Implantation flüssig ist und beispielsweise unter Druck aus dem Katheter gepreßt wird sowie nach einen gewissen Zeit am Implantationsort aushärtet.

Um die Behandlung in optimaler Weise überwachen zu können ist es zweckmäßig, das Befestigungsmittel mit Diagnosekontrastmitteln zu kennzeichnen, um die Überwachung der Operation, etwa mit Hilfe von Röntgenstrahlung oder magnetischer Resonanz, zu erleichtern. Um die Bildgebung bei der Magnetresonanztomographie nicht zu stören, sollte dabei nicht-ferromagnetisch wirksames Metall verwendet werden.

In einer vorteilhaften Weiterbildung ist das Befestigungsmittel zur Aufnahme und kontrollierten Abgabe einer vorbestimmten Menge von Medikamenten geeignet. Dadurch wird eine optimale medikamentöse Versorgung örtlich begrenzter geschädigter Areale zuverlässig gewährleistet, etwa im Falle einer Behandlung von Geschwüren oder dergleichen.

Zur Aufnahme von Medikamenten kommen dabei etwa Hohlräume in Frage, die im Befestigungsmittel selbst angeordnet sind, und die entweder mit Öffnungen versehen sind, aus denen das Medikament in kontrollierter Weise austritt, oder die, bei Verwendung eines biologisch abbaubaren Materials für das Befestigungsmittel, beim Zersetzungsprozeß des Befestigungsmittels das Medikament freigeben.

Insbesondere wenn größere Mengen an Medikamenten zur Anwendung gelangen sollen, ist es zweckmäßig , einen Medikamentenbeutel mit Hilfe des Befestigungsmittels in unmittelbarer Nähe der erkrankten Stelle anzubringen.

Der Medikamentenbeutel, der vorzugsweise aus einem biologisch abbaubaren Material besteht, kann kleine Öffnungen aufweisen, aus denen das Medikament in vorbestimmter Rate austritt.

Bei einer anderen vorteilhaften Weiterbildung weist der Katheter nach der Erfindung einen langgestreckten. Hohlkörper auf, der zumindest an seinem bei bestimmungsgemäßem Gebrauch vorderen Ende offen ist, und in dem das oder die Befestigungsmittel axialbeweglich aufgenommen ist/sind. Der Einsatz eines derartigen Katheters benötigt nur einen geringen Einführquerschnitt und ist deshalb auch bei Gefäßen mit kleinem Lumen geeignet. Nach lagerichtiger Plazierung des Katheters wird das in diesem aufgenommene Befestigungsmittel aus dem Katheter ausgetrieben und in die Gefäßwand implantiert.

Bei einer abermals anderen Ausführungsform besteht der Hohlkörper aus einem elastischen Material, so daß er beim Einführen des Katheters in das Körpergefäß im langgestreckten Zustand vorliegt, und erst nach lagerichtiger Plazierung im Körpergefäß mit seinem vorderen Abschnitt in Richtung auf die Gefäßwand aufgebogen wird. Wichtig ist dabei, daß der Innenquerschnitt des Hohlkörpers beim Aufbiegen im wesentlichen gleich bleibt, um zu verhindern, daß das Befestigungsmittel im Hohlkörper verkantet oder nicht in bestimmungsgemäßer Weise in das Körpergefäß implantiert wird.

Eine einfache, aber wirkungsvolle Möglichkeit, das Befestigungsmittel in bestimmungsgemäßer Weise in die Wandung des Hohlorgans zu implantieren, besteht darin, daß im Hohlkörper des Katheters ein Pusher axialbeweglich aufgenommen ist, der bei.bestimmungsgemäßen Einsatz am proximalen Ende des Katheters betätigbar ist und an seinem vorderen Ende mit dem Befestigungsmittel derart in Wirkverbindung steht, daß mit seiner Hilfe das Befestigungsmittel aus dem Katheter hinausschiebbar und in die Organwand implantierbar ist.

Bei einer wiederum anderen Ausführungsform ist das Befestigungsmittel schrauben- oder spiralförmig ausgebildet und besitzt an seinem hinteren Abschnitt eine Kupplung zum drehfesten, aber lösbaren Verbinden mit einem entsprechenden Kupplungsabschnitt des Pushers. Die Implantation erfolgt bei dieser Ausführungsform in der Art, daß nach lagerichtiger Plazierung des Katheters das Befestigungsmittel durch eine entsprechende Betätigung des Pushers in die Organwand hineingeschraubt und nach erfolgter Implantation vom Pusher gelöst wird.

Bei einer abermals vorteilhaften Weiterbildung ist der Hohlkörper des Katheters wenigstens über einen Teil seiner Längserstreckung in einer Hülse aufgenommen. Die Hülse dient dabei dem Schutz des Hohlkörpers und in dem Falle, daß der Hohlkörper aus einem besonders flexiblen Material besteht, zur Stabilisierung des Katheters beim Einführen in das Hohlorgan.

Bei Kathetern mit flexiblem Hohlkörper kann der Vorderabschnitt des Hohlkörpers in einfacher Weise durch ein Filament aufgebogen werden, das sich in seinem bestimmungsgemäßen Zustand am Katheter entlang erstreckt und dessen vorderes Ende mit dem Hohlkörper verbunden ist. Durch geeignete Mittel, etwa durch die vorerwähnte Hülse, wird der Katheter mit Ausnahme eines vorbestimmten Vorderabschnittes versteift, so daß nur letzterer durch Betätigen des Filaments aufbiegbar ist.

Bei einer wiederum anderen Ausführungsform verläuft das Filament im Innern des Hohlkörpers in einem zweiten Lumen in der Wend des Katheters. Das Aufbiegen des Vorderabschnittes erfolgt bei dieser Ausführungsform nach der Art eines Bowdenzugs. Das Filament kann auch aus einem Draht bestehen, wodurch eine verbesserte Stabilität erreicht wird.

Um Schädigungen des Katheters und auch Verletzungen der Gefäßwand möglichst zu vermeiden, ist der bestimmungsgemäße Beugungsbereich nach Anspruch 24 mit einer Armierung versehen, bei der es sich beispielsweise um eine dünne, flexible Metallfolie handeln kann, die in diesem Bereich im Hohlkörper des Katheters angeordnet ist. Der Katheter kann auch vollständig aus Metall, wie z.B. Nitinol, bestehen.

Bei einer abermaligen Weiterbildung steht das Befestigungsmittel mit Federmitteln in Wirkverbindung, mit deren Hilfe das Befestigungsmittel mit hoher Geschwindigkeit in die Wandung des Hohlorgans eingebracht wird und dort eine besonders zuverlässige und feste Verbindung etwa eines Dissekats an die Gefäßwandung herstellt. Die Federmittel können innerhalb des Hohlorgans im Katheter im unmittelbaren Anschluß an das Befestigungsmittel angeordnet sein. Beim Einfühen des Katheters in das Hohlorgan sind die Federmittel vorgespannt und werden nach lagerichtiger Plazierung des Katheters durch Betätigen einer geeigneten Lösevorrichtung freigegeben. Dies kann beispielsweise mittels eines Pushers erfolgen, der durch den Hohlkörper des Katheters hindurchgeführt ist. Es ist auch möglich, die Federmittel am proximalen Ende des Katheters, also außerhalb des Körpers anzubringen und die Federkräfte mit Hilfe des Pushers auf das Befestigungsmittel zu übertragen.

Anstelle der Federmittel ist auch ein elektromagnetischer Antrieb möglich, der außerhalb des Körpers angeordnet ist und das Befestigungsmittel über einen Pusher um eine vorgegebene Distanz vortreibt. Eine wieder andere Alternative sieht vor, daß das Befestigungsmittel mittels Ultraschallwellen die von einer mit dem Lumen des Hohlkörpers des Katheters in Wirkverbindung stehenden Ultraschallordnung emittiert werden, in die Wandung des Hohlorgans eingetrieben wird.

Zweckmäßigerweise sind im Hohlkörper des Katheters mehrere gleichzeitig implantierbare Befestigungsmittel aufgenommen. Diese Ausgestaltung ist insbesondere bei der Befestigung von größeren Schutzüberdeckungen bzw. eines schlauchartigen Grafts von Vorteil.

Bei einer Weiterbildung sind dabei mehrere Befestigungsmittel in entsprechenden Aufnahmeabschnitten des Vorderabschnitts des Katheterhohlkörpers angeordnet. Nach lagerichtiger Plazierung des Katheters im Hohlorgan werden die verschiedenen Aufnahmeabschnitte unabhängig voneinander in Richtung auf den jeweiligen bestimmungsgemäßen Implantationsort umgebogen und sodann die Befestigungsmittel, vorzugsweise gleichzeitig, eingetrieben.

Um einen rationellen und schonenden Eingriff zu gewährleisten, sind nach Anspruch 30 mehrere Befestigungsmittel aufeinanderfolgend im Hohlkörper des Katheters angeordnet, so daß eine sequentielle Implantation durchführbar ist. Insbesondere zur Behandlung von langgestreckten Dissekaten ist diese Ausführungsform von großem Vorteil.

Eine andere Ausgestaltung der erfindungsgemäßen Implantationsvorrichtung sieht vor, daß am Katheter ein Magazin mit mehreren Befestigungsmitteln sowie eine Ladevorrichtung angeordnet sind. Nach jeder Implantation eines Befestigungsmittels entnimmt die Ladevorrichtung ein anderes Befestigungsmittel dem Magazin und positioniert es an die Stelle im Hohlkörper, die zuvor das nunmehr implantierte Befestigungsmittel inne hatte. Auf diese Weise kann eine große Zahl von Befestigungsmitteln im Verlaufe eines einzigen Eingriffs implantiert werden.

Alternativ zum Magazin sind nach Anspruch 32 elastisch streckbare Befestigungsmittel vorgesehen, die seitlich zum Pusher im Hohlkörper des Katheters und/oder in der Hülle des Katheters aufgenommen sind, und mit Hilfe geeigneter Mittel, etwa eines koaxial um den Pusher verlaufenden Ladepushers, nach erfolgter Implantation eines ersten Befestigungsmittels, auf die Ausgangsposition des implantierten Befestigungsmittels geschoben werden, wobei sie sich selbsttätig in die bestimmungsgemäße Implantationsform bringen.

Bei einer anderen Ausführungsform steht der Hohlkörper des Katheters mit einem Zuführschlauch in Verbindung, durch den Befestigungsmittel mit Hilfe eines separaten Zuführpushers in den Katheter-Hohlkörper nachgeschoben werden können. Besonders vorteilhaft ist dabei, daß im Prinzip beliebig viele Befestigungsmittel eingebracht werden können, ohne daß der Katheter aus dem Körpergefäß entfernt werden müßte. In ähnlicher Weise kann jedoch auch der Katheter-Hohlkörper selbst als Zuführschlauch eingesetzt werden.

Um einen zufriedenstellenden Behandlungserfolg zu erzielen, ist es wichtig, daß das zur Implantation mit einem Befestigungsmittel vorgesehene Gewebe zum Zeitpunkt der Operation unter Vorspannung steht. Bei den Ausführungsbeispielen gemäß der Patentansprüche 14 bis 33 wurde diese Vorspannung insbesondere durch den umgebogenen Vorderabschnitt des Hohlkörpers gewährleistet. Um die Vorspannung zu verbessern, sehen die Ansprüche 34 und 35 zusätzliche, am Katheter angeordnete Vorrichtungen vor.

Beim Gegenstand nach Anspruch 34 handelt es sich dabei um eine Spreizhülse, die aus jeweils durch Längsschlitze voneinander getrennten Stegen besteht. Die Spreizhülse wird im gestreckten Zustand, in dem die Stege im wesentlichen parallel aneinanderliegend angeordnet sind, mit dem Katheter in das Gefäß eingeführt. Nach lagerichtiger Plazierung des Katheters wird die Spreizhülse mittels eines geeigneten Zuggliedes aufgespreizt, um das Gefäß in die gewünschte Vorspannung zu bringen. Nach erfolgter Implantation wird die Spreizhülse wieder in den gestreckten Zustand überführt und aus dem Gefäß entfernt.

Nach Anspruch 35 steht der Katheter mit einem Ballonkatheter in Wirkverbindung, der nach seiner Expansion ebenfalls arne gleichmäßige Aufdehnung des gesamten Gefäßabschnittes bewirkt. Der Implantationskatheter verläuft teilweise innerhalb der Spreizhülse bzw. des Ballonkatheters und wird dort in die bestimmungsgemäße Position gebracht. Die Implantation des Befestigungsmittels kann im Falle des Ballonkatheters dabei etwa durch eine vorbestimmte Öffnung in der Hülle des Ballonkatheters erfolgen.

Um ein Abrutschen der Katheterspitze bzw. des Befestigungsmittels an der Wandung des Hohlorgans zu verhindern, ist nach Anspruch 36 das Vorderende des Katheters angespitzt und dringt nach lagerichtiger Plazierung des Katheters um ein geringes Maß in die Gefäßwand ein, wodurch die Gefahr des Abrutschens im wesentlichen be-seitigt ist.

Sind Mittel zur Aufnahme einer Schutzüberdeckung am Katheter vorgesehen, so ist es von besonderem Vorteil, wenn diese unabhängig voneinander, etwa mittels geeigneter Positionierdrähte, gesteuert werde können, wodurch es möglich ist, die Schutzumhüllung mit einer großen Genauigkeit an dem bestimmungsgemäß Ort zu positionieren.

Die Positionierdrähte sollten dabei möglichst innerhalb gesonderter Kanäle in der Wand des Katheters angeordnet sein.

Bei einer wiederum anderen Ausführungsform weist der Katheter eine Sonde aus einem dünnen Filament auf, auf der im Einführzustand der Implantationsvorrichtung ein zumindest abschnittsweise als Hohlkörper ausgebildetes Befestigungsmittel ablösbar aufgenommen ist. Diese Ausführungsform ist besonders zweckmäßig bei sehr dünnen Gefäßquerschnitten. Auch eine solche Sonde kann mittels eines an der Sonde befestigten Filaments mit seinem Vorderabschnitt aufgebogen und somit das Befestigungsmittel in eine zur Implantation bevorzugte Ausgangsposition gebracht werden. Als Pusher für das Einbringen des Befestigungsmittels in die Gefäßwand dient dabei der koaxial um die Sonde verlaufende Katheter selbst.

Bei einer vorteilhaften Weiterbildung ist die Implantationsvorrichtung mit einer am Katheter angeordneten Ultraschallsonde zur Bilderfassung ausgestattet, die eine direkte Überwachung des Operationsbereiches erlaubt.

Anhand der beigefügten Zeichnungen sollen nachfolgend mehrere Ausführungsbeispiele der Erfindung näher erläutert werden. In schematischen Ansichten zeigen:
- Fig. 1: ein Körpergefäß mit einem Dissekat im Querschnitt,
- Fig. 2: ein mit Befestigungsmitteln an die Gefäßwand angelegtes Dissekat,
- Fig. 3: in einer vergrößerten Ausschnittansicht verschiedene Befestigungsmittel,
- Fig. 4: den Vorderabschnitt eines in ein Gefäß eingeführten Katheters einer Implantationsvorrichtung in einer Längsschnittansicht,
- Fig. 5: den Vorderabschnitt eines Katheters einer Implantationsvorrichtung in einer anderen Ausführungsform im Längsschnitt,
- Fig. 6a: einen in ein nur angedeutetes Gefäß eingeführten, nicht aufgeweiteten Ballonkatheter mit einer auf diesem aufgenommenen und Befestigungsmittel haltenden Hülle,
- Fig. 6b: in einer Ansicht wie in Fig. 6a den Ballonkatheter mit der auf diesem aufgenommenen Hülle im aufgeweiteten Zustand,
- Fig. 7: den Vorderabschnitt einer Implantationsvorrichtung in einer anderen Ausführungsform mit einem spiralförmig gewundenen Befestigungsmittel,
- Fig. 8: das Befestigungsmittel aus Fig. 7 und einen Pusher in einer vergrößerten Explosionsdarstellung,
- Fig. 9: die Kupplung des Befestigungsmittels und des Pushers in einer der Schnittlinie IX - IX in Fig. 7 entprechenden vergrößerten Querschnittansicht,
- Fig. 10: eine zur Behandlung eines Aneurysmas vorgesehene, mit Befestigungsmitteln an der Wandung eines Körpergefäßes im Bereich einer Gefäßaufzweigung angeordnete Abdeckung,
- Fig. 11: einen an einer Gefäßwand mit Hilfe eines Befestigungsmittels angebrachten Medikamentenbeutel,
- Fig. 12: den Vorderabschnitt einer Implantationsvorrichtung in einer anderen Ausführungsform mit mehreren gleichzeitig implantierbaren Befestigungsmitteln in einer teilweise geschnittenen Ansicht,
- Fig. 13: einen teilweise mit Befestigungsmitteln an eine Gefäßinnenwand angehefteten schlauchförmigen Graft im bestimmungsgemäßen Zustand in einem Längsschnitt,
- Fig. 14: eine mit mehreren Befestigungsmitteln bestückte und mit einer Feder zur Unterstützung der Implantation versehene Implantationsvorrichtung im gestreckten Zustand in einem Längsschnitt,
- Fig. 15: eine als langgestrecktes Filament ausgebildete Sonde zur Implantation von Befestigungsmitteln und zugehörende Befestigungsmittel im Längsschnitt,
- Fig. 16: eine in einem nur angedeuteten Gefäß aufgenommene mit einer Spreizhülse sowie mit einem Magazin zur sequentiellen Implantation von Befestigungsmitteln versehene Implantationsvorrichtung,
- Fig. 17: eine in einem Gefäß aufgenommene mit einem Zuführschlauch zum Einbringen von Befestigungsmitteln versehene Implantationsvorrichtung,
- Fig. 18: eine Implantationsvorrichtung mit einem seitlich angeordneten, elastisch streckbaren Ersatz-Befestigungsmittel und
- Fig. 19: eine in einem Ballonkatheter, der in einem Gefäß aufgenommen ist, angeordnete Implantationsvorrichtung.

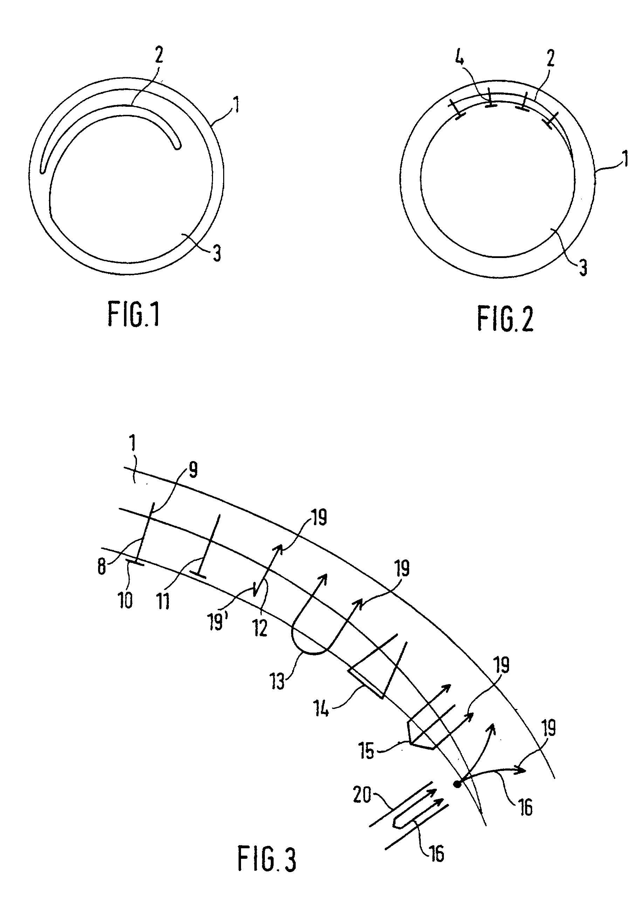

Das in Fig. 1 gezeigte Körpergefäß 1 weist ein sogenanntes Dissekat 2 auf, also ein in das Lumen 3 des Körpergefäßes 1 teilweise hineinragendes Gewebeteil. Derartige Dissekate entstehen häufig nach einer vorangegangenen angioplastischen Behandlung des Körpergefäßes mittels Ballonkathetern. Bei der Behandlung des Dissekats 2 mit der erfindungsgemäßen Implantationsvorrichtung wird das Dissekat 2 an die Innenwand des Körpergefäßes 1 angedrückt und mit Hilfe eines oder mehrerer Befestigungsmittel 4 an die Wandung des Körpergefäßes 1 angeheftet.

In Fig. 3 sind verschiedene Befestigungsmittel 8, 11 bis 16 in ihrem bestimmungsgemäßen Implantationszustand in der Wandung eines Körpergefäßes 1 gezeigt. Ein einfaches, jedoch in den meisten Fällen ausreichendes Befestigungsmittel stellt ein Nagel 8 dar, dessen Schaft 9 eine der Dicke der Gefäßwand angepaßte Länge besitzt. Um etwaig an dem aus der Gefäßwand vorstehenden Nagelkopf 10 sich bildende Stenosen zu vermeiden, ist es zweckmäßig, den Nagel 10 bei der Implantation derart weit in die Wand des Körpergefäßes 1 hineinzutreiben, daß der Nagelkopf nicht mehr in das Lumen des Körpergefäßes 1 hineinragt, wie dies am Beispiel des Nagels 11 gezeigt ist. Nach einer gewissen Zeit wird der Nagel 11 auch an seinem Kopfende von Gefäßgewebe überdeckt und stellt somit im Hinblick auf mögliche Stenosen keine Gefahrenquelle mehr dar.

Zur Herstellung einer besonders festen Verbindung weist der Nagel 12 sowohl an seiner Schaftspitze wie auch an seinem Kopfteil Widerhaken 19, 19' auf. Für unterschiedliche Anwendungen können die Befestigungsmittel auch als Krampen ausgebildet sein. Die Krampe 13 ist mit U-förmig zueinander gebogenen Schenkeln versehen, die Krampe 14 weist zwei an einem im bestimmungsgemäßen Zustand parallel zur Innenwand des Körpergefäßes 1 verlaufenden Steg befestigte und sich einander zuneigende Schenkel auf. Eine besonders feste Verbindung wird durch die dreischenklige Krampe 15 hergestellt, die - ebenso wie die U-förmig gebogene Krampe 13 - zusätzlich mit Widerhaken 19 ausgerüstet sein kann.

Ein besonders zweckmäßiges Befestigungsmittel stellt die mit zwei elastischen Schenkeln versehene Krampe 16 dar: Beim Einführen eines Katheters 20 sind diese im Hohlkörper des Katheters aufgenommen, wobei die Schenkel der Krampe 16 eng aneinanderliegen. Bei der Implantation, das heißt, wenn die Krampe 16 aus dem Katheter 20 ausgetrieben wird und in die Gefäßwandung eindringt, spreizen sich die beiden Schenkel zum bestimmungsgemäßen Implantationszustand der Krampe 16 auf.

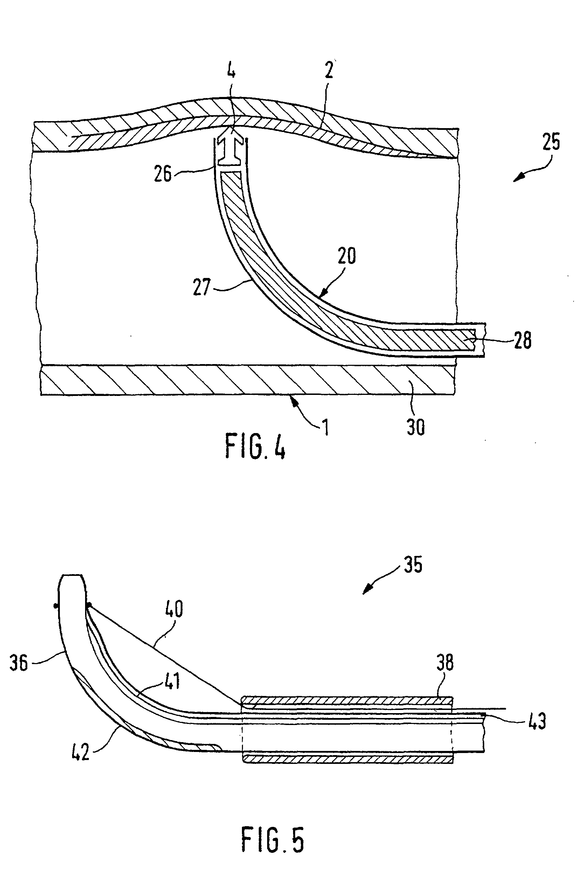

Fig. 4 zeigt eine Implantationsvorrichtung 25 im bestimmungsgemäßen Zustand zur Behandlung eines Dissekats 2 in der Wandung eines Körpergefäßes 1. Die Implantationsvorrichtung 25 besteht aus einem Katheter 20 und einem Befestigungsmittel 4. Der Katheter 20 weist einen langgestreckten, an seinem distalen Ende 26 offenen Hohlkörper 27 sowie einen durch diesen hindurchgeführten, mit dem Befestigungsmittel 4 in Wirkverbindung stehenden Pusher 28 auf. Der Hohlkörper 27 ist in seinem vorderen Abschnitt derart aufgebogen, daß das distale Ende 26 des Hohlkörpers 27 im wesentlichen senkrecht zur Längsachse des Körpergefäßes 1 angeordnet ist. Sich an einem Wandabschnitt 30 des Körpergefäßes 1 abstützend drückt der Hohlkörper 27 mit seinem distalen Ende 26 gegen die Wandung des Körpergefäßes 1 im Bereich des Dissekats 2 und hält das Gefäß 1 somit in einer für die Implantation vorteilhaften Vorspannung. Die Implantation des Befestigungsmittels 4 erfolgt nach lagerichtiger Plazierung des Katheters 20 durch Vorschieben des Pushers 28 im Hohlkörper 27.

Während der Katheter 20 gemäß Fig. 4 einen im wesentlichen elastischen Hohlkörper aufweist und mit Hilfe eines - hier nicht gezeigten - Führungsdrahtes in einem gestreckten Zustand eingeführt wird, sich nach lagerichtiger Plazierung im Körpergefäß jedoch selbsttätig in die bestimmungsgemäße, gekrümmte Form aufrichtet, besitzt der in Fig. 5 dargestellte Katheter 35 einen Hohlkörper 36 aus einem flexiblen Material.

Zur Stabilisierung des Hohlkörpers 36 während des Einführens des Katheters 35 in ein Körpergefäß 1 ist der Hohlkörper 36 über den Großteil seiner Längserstreckung von einer Hülse 38 umgeben. An seinem distalen Ende ist der Hohlkörper 36 mit einem Filament 40 verbunden, mit dessen Hilfe der vordere Abschnitt des Hohlkörpers 36 zur bestimmungsgemäßen Implantationsposition aufgebogen wird. Das Filament 40 ist - wie auch der Hohlkörper 36 - axialbeweglich in der Hülse 38 aufgenommen. Nach dem Einführen des Katheters 35 in ein Körpergefäß wird die Hülse um eine vorbestimmte Distanz vom distalen Ende des Hohlkörpers 36 zurückgezogen, um einen für die entsprechende Anwendung geeigneten Vorderabschnitt des Hohlkörpers 36 zum Aufbiegen mit Hilfe des Filaments 40 freizugeben. Eine Armierung 42 verhindert, daß das Befestigungsmittel und/oder der Pusher beim Vorschieben im Hohlkörper 36 dessen Wandung durchdringt. Anstelle des außen am Hohlkörper 36 innerhalb der Hülse 38 angeordneten Filaments 40 ist auch ein innenseitig im Hohlkörper 36 in einem gesonderten Lumen 43 verlaufendes Filament 41 einsetzbar, das ebenfalls am Vorderabschnitt des Hohlkörpers 36 befestigt ist und diesen nach der Art eines Bowdenzugs in seine bestimmungsgemäße Position aufbiegt.

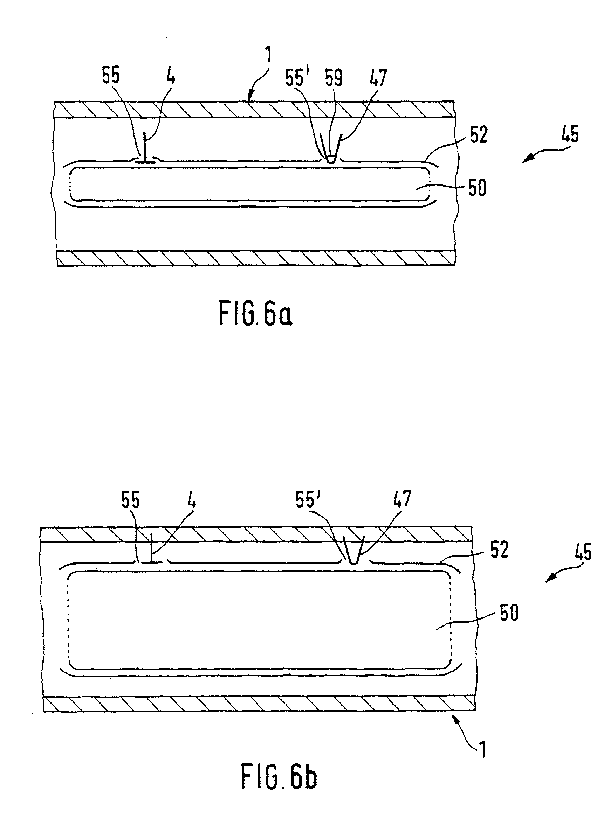

Die Fig. 6a und b zeigen eine andere Ausführungsform einer erfindungsgemäßen Implantationsvorrichtung 45. Dabei sind die Befestigungsmittel 4, 47 radial außenseitig an der Außenwand eines Ballonkatheters 50 angeordnet. Der Ballonkatheter 50 ist von einer flexiblen Hülle 52 umschlossen. Mit ihren - der Wandung des Körpergefäßes 1 zugewandten - Implantationsabschnitten durchdringen die Befestigungsmittel 4, 47 jeweils in der Hülle 52 angeordnete Öffnungen 55, 55'. Im - in Fig. 6a gezeigten - nicht aufgeweiteten Zustand des Ballonkatheters 50 ist der Querschnitt der Hüllenöffnung 55 kleiner als der Querschnitt des Kopfs des als Nagel ausgebildeten Befestigungsmittels 4. Somit wird das Befestigungsmittel 4 durch die Hülle radialfest am Ballonkatheter gehalten. Das als Krampe ausgebildete Befestigungsmittel 47 wird durch ein stegartiges Haltefilament 59 der Hülle 52 ebenfalls radialfest am Ballonkatheter 50 gehalten.

Im expandierten, in Fig. 6b gezeigten, Zustand des Ballonkatheters 50 ist die Hüllenöffnung 55 derart aufgeweitet, daß das Befestigungsmittel 4 auch mit seinem Kopf freigegeben ist. Gleichzeitig wird das Befestigungsmittel 4 infolge der Expansion des Ballonkatheters 50 mit seinem Implantationsabschnitt in die Wand des Körpergefäßes 1 eingetrieben. Beim Aufdehnen des Ballonkatheters 50 wird zudem das stegartige Haltefilament 59, welches das Befestigungsmittel 47 während des Einführvorgangs des Katheters gehalten hatte, zerrissen und somit auch das Befestigungsmittel 47 freigegeben, das in prinzipiell gleicher Weise wie das Befestigungsmittel 4 in die Wandung des Körpergefäßes 1 implantiert wird. Nach erfolgter Implantation wird der Ballonkatheter auf seinen Einführzustand (mit kleinem Querschnitt) gebracht und wieder aus dem Körpergefäß entfernt.

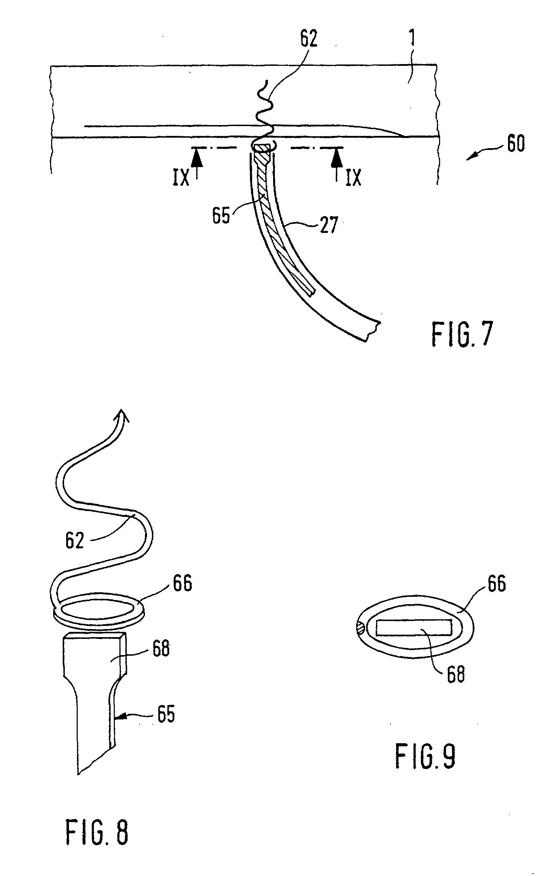

Die Fig. 7 bis 9 zeigen eine Implantationsvorrichtung 60 in einer wieder anderen Ausführungsform mit einem spiralförmig gewundenen Befestigungsmittel 62. Mit einem derartigen Befestigungsmittel 62 läßt sich eine besonders zuverlässige und haltbare Verbindung herstellen. Implantiert wird das Befestigungsmittel 62 mittels eines Katheters, in dessen Hohlkörper 63 ein Pusher 65 axial- und drehbeweglich aufgenommmen ist. Das Befestigungsmittel 62 weist an seinem proximalen Ende einen Kupplungsabschnitt 66 auf, der mit einer Kupplungsaufnahme 68 des Pushers drehfest, aber lösbar verbunden ist. Durch eine geeignete Drehbewegung des Pushers wird das Befestigungsmittel 62 in die Wandung des Körpergefäßes 1 eingeschraubt. Wie Fig. 9 verdeutlicht, wird die Übertragung des Drehmoments durch geeignete Geometrien des Kupplungsabschnitts 66 und der Kupplungsaufnahme 68 vermittelt, indem nämlich die Kupplungsaufnahme 68 mit ihrem rechteckigen Querschnitt in den als eliptische oder ovale Schlaufe geformten Kupplungsabschnitt 66 eingreift.

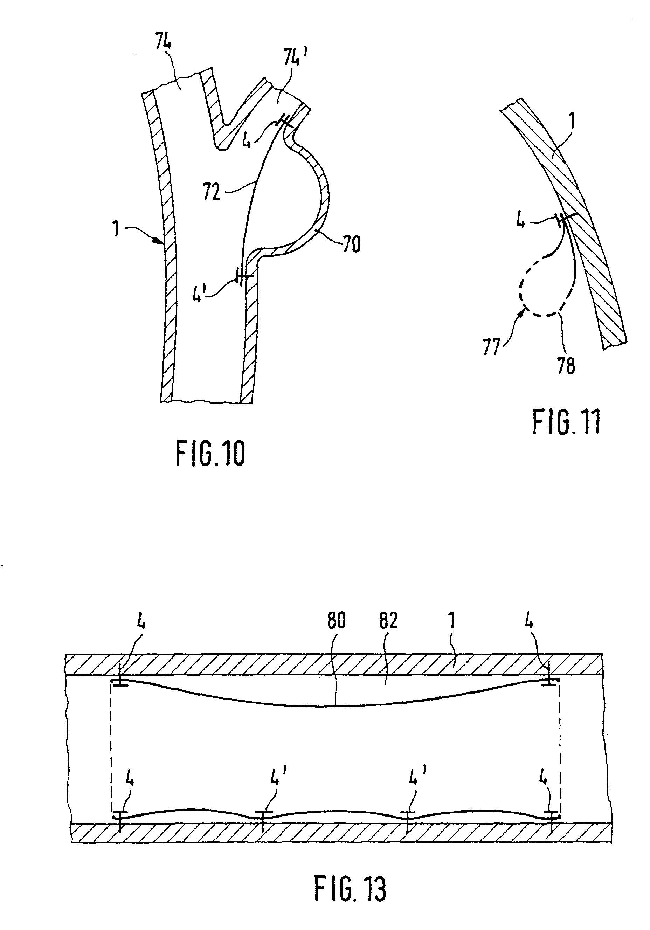

Fig. 10 zeigt ein Körpergefäß 1 im Bereich einer Gefäßaufzweigung, bei dem sich ein Aneurysma 70 ausgebildet hat. Die Behandlung des Aneurysmas mittels einer erfindungsgemäßen Implantationsvorrichtung erfolgt in der Weise, daß eine der Größe des geschädigten Gewebeareals angepaßte Überdeckung 72 die etwa aus einem Textilgestricke, eines Gewebeteils oder einer Patch-Plastik bestehen kann, während des Einführens der Implantationsvorrichtung in das Körpergefäß 1 etwa im Bereich zwischen der Hülle 38 und dem Hohlkörper 36 eines Katheters 35 aufgenommen ist und am Implantationsort nochmit Hilfe einer geeigneten Vorrichtung - was unten näher beschrieben wird - auf das erkrankte Areal aufgelegt wird. Mit Hilfe von Befestigungsmitteln 4, 4' wird die Überdeckung 72 dann in gesunden Gewebebereichen an die Gefäßwand angeheftet.

Während bei konventionellen Behandlungsmethoden, bei denen etwa eine einer Gefäßaufzweigung angepaßte Endoprothese implantiert wird, eine erhebliche Verengung des Gefäßquerschnitts unvermeidlich ist und darüber hinaus auch weite, gesunde Bereiche des Körpergefäßes mit eher schädlicher Folge für diese Areale mittherapiert werden, zeigt Fig. 10 besonders deutlich, daß über den unmittelbar geschädigten Bereich 70 hinaus kaum Abschnitte des Körpergefäßes 1 von der Therapie betroffen sind; ferner, daß eine Gefäßverengung durch die Überdeckung lediglich in einem der Zweige 74, 74' des sich aufzweigenden Körpergefäßes 1 besteht, und auch dort nur in einem im Vergleich zur Behandlung mit einem konventionellen Stent stark reduziertem Maße.

Fig. 11 zeigt die Befestigung eines medikamentengefüllten Beutels 77 mit einem Befestigungsmittel 4 an der Gefäßwand 1. Der Beutel 77 weist Öffnungen 78 auf, durch die im Beutel 77 befindliches Medikament in kontrollierter Rate an die das Körpergefäß 1 durchströmende Körperflüssigkeit abgegeben wird. Besonders vorteilhaft ist die Befestigung eines derartigen Beutels 77 in unmittelbarer Nähe einer stark lokalisierten Gewebserkrankung oder in einer einen Tumor versorgenden Arterie.

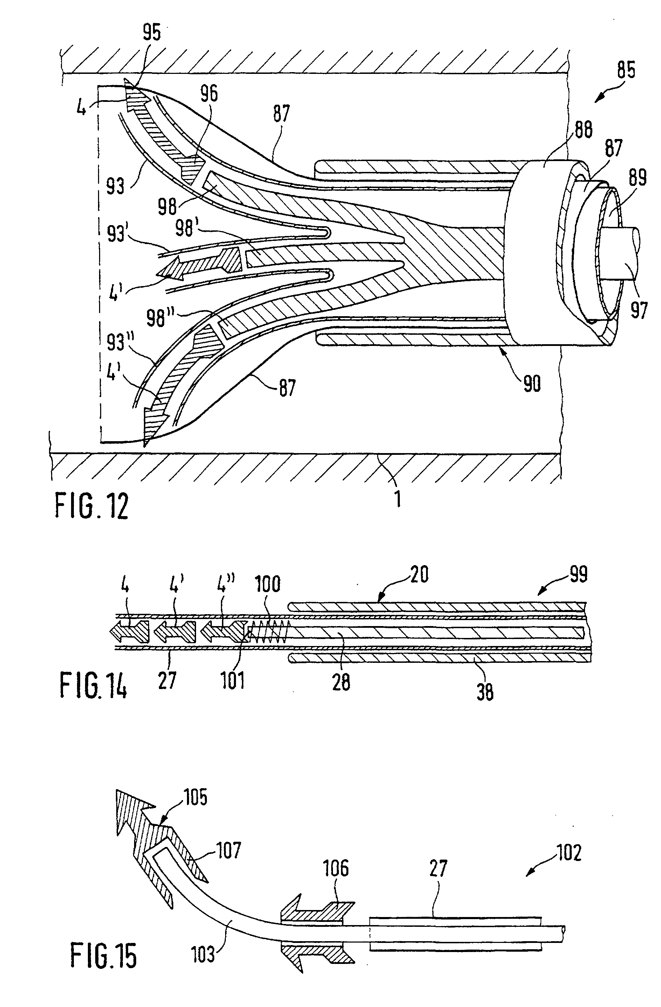

Fig. 12 zeigt eine Implantationsvorrichtung 85 zur Implantation und Befestigung eines schlauchförmigen Grafts 87 in das Körpergefäß 1. Beim Einführen der Implantationsvorrichtung 85 ist der vorzugsweise aus einem elastischen Material bestehende Graft 87 zwischen einer Hülse 88 und dem Hohlkörper 89 des Katheters 90 aufgenommen. Nach lagerichtiger Plazierung des Katheters 90 wird die Hülse 88 um eine vorbestimmte Distanz zurückgezogen, woraufhin sich das Graft 87 in seine bestimmungsgemäße Implantationslage aufdehnt. Die Aufdehnung erfolgt dabei in der Weise, daß das Graft 87, der beim Einführen der Implantationsvorrichtung 85 zwischen der Hülse 88 und dem Hohlkörper 89 des Katheters 90 in einem komprimierten oder eingefalteten Zustand vorliegt, durch das Zurückziehen der Hülse freigegeben und entweder selbsttätig oder durch Zuhilfenahme des Hohlkörpers 89 des Katheters 90 in seinen bestimmungsgemäßen Aufweitungszustand gelangt.

Zu diesem Zweck weist der Hohlkörper 89 mehrere, im Ausführungsbeispiel drei, Aufnahmeabschnitte 93, 93', 93'' auf, die beim Einführen des Katheters 90 in das Körpergefäß 1 parallel zueinander verlaufen, im Implantationszustand des Katheters jedoch in Richtung auf die Wandung des Körgefäßes 1 umgebogen sind, wobei die Aufnahmeabschnitte 93, 93', 93'' in jeweils unterschiedliche Richtungen weisen. Das Umbiegen der Aufnahmeabschnitte 93, 93', 93'' erfolgt dabei entweder dadurch, daß die Aufnahmeabschnitte 93, 93', 93'' als gekrümmte, jedoch elastische Hohlkörper ausgebildet sind, die beim Einführen der Implantationsvorrichtung 85 unter Spannung parallel zueinander gehalten werden, und sich nach Zurückziehen der Hülse 88 in ihren gekrümmten Ursprungszustand selbsttätig aufbiegen. Alternativ hierzu kann jedoch auch eine Vorrichtung ähnlich der in Fig. 5 gezeigten zum Einsatz kommen, bei der Filamente zum Aufbiegen der Aufnahmeabschnitte verwendet werden.

In den Aufnahmeabschnitten 93, 93', 93'' sind im Einführzustand des Katheters 90 Befestigungsmittel 4, 4', 4'' aufgenommen, deren Aufgabe zum einen das Halten des Grafts 87 während des Einführens des Katheters 90 in das Körpergefäß 1 ist, wobei dieses Halten etwa dadurch erfolgen kann, da die Befestigungsmittel 4, 4', 4'' mit einem Teil ihrer Implantationsabschnitte das Craft durchdringen. Zum anderen dienen die Befestigungsmittel 4, 4', 4'' dazu, das Graft 87 im implantierten Zustand an der Wandung des Körpergefäßes 1 zu befestigen, indem die Befestigungsmittel 4, 4', 4'' mit ihren Implantationsabschnitten 95 das Ggraft 87 und teilweise die Wandung des Körpergefäßes 1 durchdringen und mit ihren Halteabschnitten 96 das Graft 87 an der Gefäßwand fixieren. Die Implantation der Befestigungsmittel 4, 4', 4'' geschieht in schon bekannter Weise jeweils mittels eines Pushers 97, der sich im Ausführungsbeispiel an seinem distalen Ende in eine der Anzahl der Aufnahmeabschnitte 98, 98', 98'' entsprechende Zahl von Vorderabschnitten 99, 99', 99'' aufteilt und somit bei bestimmungsgemäßem Gebrauch die Befestigungsmittel 4, 4', 4'' gleichzeitig vortreibt. Alternativ zum sich verzweigenden Pusher 97 kann jedoch auch ein Bündel von unabhängig voneinander betätigbaren Pushern im Hohlkörper 88 angeordnet sein, von denen jeweils ein Pusher zur Implantation eines Befestigungsmittels dient.

Fig. 13 zeigt eine Anwendungsmöglichkeit einer erfindungsgemäßen Implantationsvorrichtung, bei der die Befestigungsmittel 4, 4' zur Befestigung eines schlauchförmigen Grafts 80 eingesetzt werden. Das Graft wird dabei zunächst etwa nach der vorbeschriebenen Methode (Fig. 12) an seinen distalen sowie proximalen Enden mittels Befestigungsmitteln 4, 4' bfestigt. In der Folge wird das Graft 80 im Bereich zwischen den Enden jedoch häufig durch die Sogwirkung der das Körpergefäß 1 durchströmenden Körperflüssigkeit in das Innere des Körpergefäßes 1 hineingezogen. Hierdurch besteht die Gefahr eines Verschlusses mit im Bereich 82 zwischen dem Stentgraft und der Wandung des Körpergefäßes 1. Um dies zu vermeiden, wird das Graft 80 im Bereich zwischen den Enden mittels einer geeigneten Zahl von Befestigungsmitteln 4, 4' an die Wandung des Körpergefäßes 1 angeheftet.

Die in Fig. 14 mit einem Katheter 20 in gestrecktem Zustand gezeigte Implantationsvorrichtung 99 weist mehrere, im Hohlkörper 27 des Katheters 20 hintereinander angeordnete Befestigungsmittel 4 auf, die zur sequentiellen Implantation bestimmt sind. Auch bei diesem Ausführungsbeispiel wird der Hohlkörper 27 durch eine Hülse 38 gestützt und die Befestigungsmittel werden mit Hilfe eines Pushers 28 vorgetrieben. Angrenzend an das dem Pusher 28 nächstliegende Befestigungmittel 4'' ist eine Spiralfeder 100 angeordnet, die beim Einführen des Katheters von einer am Pusher 28 vorstehenden Nase 101 im gespannten Zustand gehalten ist. Nach Erreichen der bestimmungsgemäßen Implantationsposition der Implantationsvorrichtung 99 wird die Feder 100 durch Drehen des Pushers freigegeben, wodurch die Feder sich entspannt und das Befestigungsmittel beaufschlagt. Dadurch wird das Eintreiben der Befestigungsmittel 4 in die Innenwand des Körpergefäßes bewirkt.

Im Unterschied zu den vorangegangenen Ausführungsbeispielen, bei denen die Befestigungsmittel im Innern des Hohlkörpers 27 angeordnet waren, dient bei der in Fig. 15 gezeigten Implantationsvorrichtung 102 der Hohlkörper 27 zur axialbeweglichen Aufnahme einer als langgestrecktes Filament ausgebildeten Sonde 103, die zur Implantation spezieller Befestigungsmittel 105, 106 bestimmt ist. Das Befestigungsmittel 105 weist einen mit einer Ausnehmung versehenen Hinterabschnitt 107 auf, mit dem das Befestigungsmittel 105 auf dem Vorderabschnitt der Sonde 103 formschlüssig, aber ablösbar aufgenommen ist. Zur Implantation des Befestigungsmittels 105 genügt eine entsprechende axiale Betätigung der Sonde. Bei der Implantation des Befestigungsmittels 106 dagegen, das als Hohlkörper ausgebildet und axialbeweglich auf der Sonde 103 aufgenommen ist, dient der Hohlkörper 27 als Pusher, der entlang der Sonde 103 vorgeschoben wird und auf diese Weise das Befestigungsmittel 106 vorschiebt.

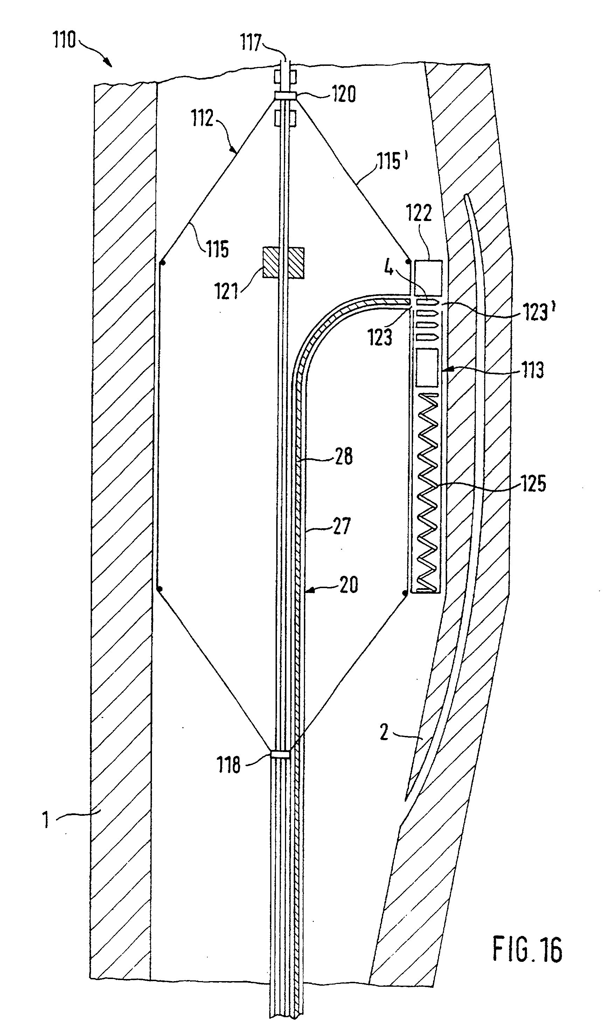

In Fig. 16 ist eine Implantationsvorrichtung 110 im bestimmungsgemäßen Zustand zur Behandlung eines Dissekats 2 in einem Körpergefäß 1 gezeigt, die mit einer Spreizhülse 112 und einem Magazin 113 für Befestigungsmittel 4 vesehen ist.

Die Spreizhülse 112 dient zur Aufweitung des Körpergefäßes 1 vor und während der Implantation. Sie besteht aus mehreren, in Umfangsrichtung unter gleichen Winkeln voneinander beabstandeten Stegen 115, 115', die jeweils aus drei langgestreckten, im wesentlichen formstabilen und in Anlenkpunkten miteinander verbundenen Abschnitten aufgebaut sind. Mit dem distalen Ende 117 der Spreizhülse 112 ist ein sich durch die Spreizhülse 112 hindurcherstreckendes und über deren proximalem Ende 118 hinausragendes Zugmittel 120 fest verbunden, mittels dessen bei im übrigen festgehaltener Spreizhülse 112 ein Aufspreizen der Stege 115, 115' in radialer Richtung durch Zugkraft bewirkt wird. Die Stege 115, 115' halten auf diese Weise das Körpergefäß 1 unter einer für die Implantation gewünschten Vorspannung.

Am Steg 115' ist radialaußenseitig ein Magazin 113 befestigt, in dem mehrere zur sequentiellen Implantation vorgesehene Befestigungsmittel 4 längs einer Linie nebeneinander angeordnet sind. Das Gehäuse 122 des Magazins 113 weist an seinem vorderen Abschnitt zwei miteinander fluchtende Öffnungen 123, 123' auf, deren Durchmesser dem Querschnitt der Befestigungsmittel 4 angepaßt sind. Mit Hilfe eines Federmittels 125 wird jeweils ein Befestigungsmittel in eine Position zwischen den Öffnungen gebracht. Die Implantation dieses Befestigungsmittels 4 erfolgt in bekannter Weise durch Betätigen eines Pushers 28, der durch den an der Öffnung 123 im Magazin einmündenden Hohlkörper 27 eines Katheters 20 geführt ist. Nach erfolgter Implantation des ersten Befestigungsmittels 4 wird der Pusher 28 aus dem Magazin zurückgezogen, worauf ein nächstes Befestigungsmittel 4 von dem Federmittel 125 in die zur Implantation geeignete Ausgangsposition gebracht wird. Eine innerhalb der Spreizhülse 112 angeordnete Ultraschallsonde 121 dient zur diagnostischen Bildüberwachung des Operationsvorgangs.

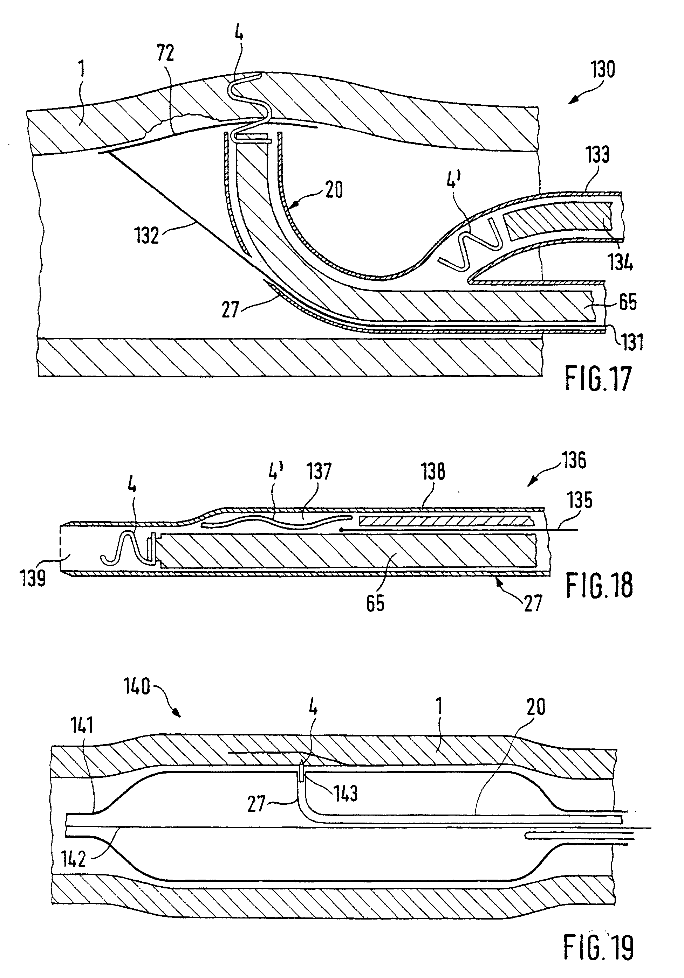

Die in Fig. 17 gezeigte Implantationsvorrichtung 130 besitzt die Möglichkeit, nach erfolgter Implantation eines Befestigungsmittels 4- welches hier wiederum, wie in Fig. 7 in einer spiralig gewundenen Ausführungsform gezeigt ist - ein nachfolgendes Befestigungsmittel 4' im Hohlkörper 27 des Katheters 20 zu plazieren, ohne daß der Katheter 20 dabei aus dem Körpergefäß 1 entfernt werden müßte. In einem Zuführschlauch 133, dessen Lumen mit dem Lumen des Hohlkörpers 27 verbunden ist, sind ein oder mehrere Befestigungsmittel 4' angeordnet, die mittels eines Zuführpushers 134 im Zuführschlauch 133 vorschiebbar sind. Nach erfolgter Implantation des Befestigungsmittels 4 wird der Implantationspusher 65 bis über die Verbindungsstelle des Zuführschlauchs 133 mit dem Hohlkörper 27 hinaus zurückgezogen. Mit Hilfe des Zuführpushers 134 wird sodann ein Befestigungsmittel 4' in den Hohlkörper 27 eingebracht. Nach dem Zurückziehen des Zuführpushers 134 steht das Befestigungsmittel 4' zur Implantation mit Hilfe des Implantationspushers 65 zur Verfügung.

In Fig. 17 ist auch ein Spanndraht 132 gezeigt, der zum Aufspannen einer Schutzüberdeckung 72 geeignet ist. Der Spanndraht 132 erstreckt sich vom Pusher 65, durch die Trennwand 131 getrennt, durch den Hohlkörper 27 hindurch und wird vom proximalen Ende des Katheters 20 betätigt. Bei der Implantation wird die Schutzüberdeckung 72 vom Spanndraht 132 gegen die Gefäßwand gedrückt, und somit während der Befestigung der Überdeckung 72 durch Befestigungsmittel in einer lagerichtigen Position gehalten.

Fig. 18 zeigt eine abermals andere Ausführungsform 136 einer Implantationsvorrichtung, bei der die Befestigungsmittel 4, 4' aus einem hochflexiblen Material gefertigt sind. Seitlich oder koaxial zum Implantationspusher 65 ist bei dieser Ausführungsform in einer Aufnahme 137 des Hohlkörpers 27 ein Befestigungsmittel 4' in einem stark gestreckten Zustand untergebracht. Ähnlich wie bei der Implantationsvorrichtung 130 wird auch bei der Ausführungsform 136 nach erfolgter Implantation des Befestigungsmittels 4 der Pusher soweit zurückgezogen, daß das Befestigungsmittel 4' mittels eines weiteren Pushers 138 in den eigentlichen Implantationskanal 139 des Hohlkörpers 27 verschiebbar ist. Dort nimmt das Befestigungsmittel 4' selbsttätig seine bestimmungsgemäße Implantationsform an und kann mittels des Implantationspushers 65 implantiert werden. Auch hier ist der Implantationskanal 135 von der Aufnahme 137 durch eine Trennwand 135 abgetrennt, die lediglich im unmittelbaren Zuführungsbereich für das Befestigungsmittel 4', also am distalen Ende der Aufnahme 137, unterbrochen ist. Durch die Trennwand 135 wird gewährleistet, daß sich Implantationspusher 65 und Zuführpusher 138 nicht gegenseitig behindern. Selbstverständlich können auch mehrere Befestigungsmittel 4' hintereinanderliegend in der Aufnahme 137 des Hohlkörpers 27 angeordnet sein.

Fig. 19 schließlich zeigt eine Implantationsvorrichtung 140, bei der die notwendige Vorspannung des Körpergefäßes 1 mittels eines Ballonkatheters 141 erfolgt. Der Ballonkatheter 141 wird mit Hilfe eines Führungsdrahtes 142 im Körpergefäß 1 plaziert und dann in seinen bestimmungsgemäßen Aufweitzustand gebracht. Der Hohlkörper 27 des Implantationskatheters 20 erstreckt sich teilweise durch den Ballonkatheter 141 hindurch und mündet an einer vorbestimmten Öffnung 143 der Außenhülle des Ballonkatheters 141, aus der heraus auch die Implantation des Befestigungsmittels 4 in die Wandung des Körpergefäßes 1 - in schon beschriebener Weise - erfolgt.

## Patentansprüche

1. Implantationsvorrichtung zur Behandlung von im Bereich der Innenwandung von Hohlorganen verletztem oder erkranktem Gewebe, insbesondere Implantationsvorrichtung zur Behandlung einer Dissektion in Körpergefäßen,
mit einem Katheter (20,35,90) und wenigstens einem mit diesem Katheters (20,35,90) verbundenen Befestigungsmittel (4, 8,1 1-16, 47, 62),
wobei der Katheter (20,35,90) einen langgestreckten, an zumindest seinem bei bestimmungsgemäßen Gebrauch vorderen Ende offenen Hohlkörper aufweist, in dem jedes Befestigungsmittel (4, 8, 11-16, 47, 62) beim Einführen der lmplantationsvorrichtung (25, 45, 60, 85, 99, 102, 110, 130, 136, 140) in das Körpergefäß (1) axialbeweglich aufgenommen ist,
**dadurch gekennzeichnet, daß**
der Hohlkörper (27, 36, 63) des Katheters (20, 35, 90) an seinem vorderen Ende aufgebogen ist und hierdurch jedes Befestigungsmittel (4, 8, 11-16, 47, 62) nach lagerichtiger Plazierung des Katheters (20, 35, 90) im Hohlorgan (1) mit zumindest einem Implantationsabschnitt (9.96) in die Wandung des Hohlorgans (1) jeweils gezielt implantierbar und anschließend von dem Katheter (20, 35, 90) lösbar ist

2. Imptantationsvorrichtung nach Anspruch 1, **dadurch gekennzeichnet, daß** der Katheter (20, 35, 90) Mittel (132) zum Aufnehmen und Positionieren einer Schutzüberdeckung (72) für das betroffene Gewebe aufweist, die nach lagerichtiger Plazierung des Katheters (20, 35, 90) im Hohlorgan (1) vom Katheter (20, 35, 90) ablösbar und mittels wenigstens eines Befestigungsmittels (4, 8, 11-16, 47, 62) an der Innenwandung des Hohlorgans (1) befestigbar ist.

3. Implantationsvorrichtung nach Anspruch 2, **dadurch gekennzeichnet, daß** es sich bei der Schutzüberdeckung (72) um ein dem geschädigten Gewebeareal angepaßtes textiles Gewebeteil oder um eine - vorzugsweise poröse - Kunststofffolie handelt.

4. Implantationsvorrichtung nach Anspruch 3, **dadurch gekennzeichnet daß** es sich bei der Textilstruktur um einen schlauchförmigen Graft (80, 87) handelt.

5. Implantationsvorrichtung nach einem der Ansprüche 1 bis 4, **dadurch gekennzeichnet, daß** als Befestigungsmittel ein Nagel (4, 8, 11) vorgesehen ist, dessen Schaft (9) den Implantationsabschnitt des Befestigungsmittels (4, 8, 11-16, 47, 62) bildet.

6. Implantationsvorrichtung nach einem der Ansprüche 1 bis 4, **dadurch gekennzeichnet daß** der Implantationsabschnit (9, 95) des Befestigungsmittels (4, 8, 11-16, 47, 62) als eine wenigstens zwei Schenkel aufweisende Krampe (13-16, 47) ausgebildet ist, welche Schenkel jeweils U- oder V- förmig zueinander angeordnet sind.

7. Implantationsvorrichtung nach einem der Ansprüche 1 bis 6, **dadurch gekennzeichnet, daß** der Implantationsabschnitt (9. 95) des Befestigungsmittels (4,8.11-16,47,62) aus wenigstens zwei elastischen an jeweils einem Ende miteinander verbundenen Schenkeln besteht, die in der bestimmungsgemäßen Implantationslage des Befestigungsmittels (4, 8, 11-16, 47, 62) jeweils im wesentlichen V-fömlig zueinander angeordnet sind.

8. Implantationsvorrichtung nach einem der Ansprüche 1 bis **dadurch gekennzeichnet, daß** der lmplantationsabschnifl (9, 95) des Befestigungsmittels (4, 8, 11-16, 47, 62) mit Widerhaken (19, 19') versehen ist.

9. Implantationsvorrichtung nach einem der Ansprüche 1 bis 8, **dadurch gekennzeichnet, daß** das Befestigungsmittel (4, 8, 11-16, 47, 62) aus einem biologisch abbaubaren Material besteht.

10. Implantationsvorrichtung nach einem der Ansprüche 1 bis 9, **dadurch gekennzeichnet, daß** das Befestigungsmittel (4, 8, 11-16, 47, 62) aus einem elastischen Material, etwa Nitinol besteht.

11. Implantationsvorrichtung nach einem der Ansprüche 1 bis 4 **dadurch gekennzeichnet, daß** für das Befestigungsmittel (4, 8, 11-16, 47, 62) ein bei der Implantation flüssiges, jedoch innerhalb einer vorgegebenen Zeitdauer aushärtendes Material verwendet wird.

12. Implantationsvorrichtung nach einem der Ansprüche 1 bis 11, **dadurch gekennzeichnet, daß** das Befestigungsmittel (4, 8, 11-16, 47, 62) mit einem Diagnosekontrastmittel versehen oder aus einem in einem gewählten Diagnoseverfahren kontrastgebendem Material besteht.

13. Implantationsvorrichtung nach einem der Ansprüche 1 bis 12, **dadurch gekennzeichnet daß** das Befestigungsmittel (4,8,11-16,47,62) zur Aufnahme und kontrollierten Abgabe einer vorbestimmten Menge von Medikamenten geeignet ist.

14. Implantationsvorrichtung nach Anspruch 13, **dadurch gekennzeichnet, daß** das Befestigungsmittel (4, 8, 11-16, 47, 62) mit einem Hohlraum zur Aufnahme einer vorbestimmten Menge von Medikamenten versehen ist.

15. Implantationsvorrichtung nach Anspruch 14, **dadurch gekennzeichnet, daß** das Befestiaunosmittel (4, 8, 11-16, 47, 62) biologisch abbaubar ist und die Medikamentenfreisetzung bei der Zersetzung des Befestigungsmittels erfolgt.

16. Implantationsvorrichtung nach Anspruch 13, **dadurch gekennzeichnet, daß** zur Aufnahme von Medikamenten wenigstens ein mit einem Kopfabschnitt (10) des Befestigungsmittels (4, 8, 11-16, 47, 62) verbundener Medikamentenbeutel (77) vorgesehen ist, der mit Öffnungen (78) zur Abgabe des im Medikamentenbeutel (77) speicherbaren Medikaments in vorbestimmter Rate versehen ist.

17. Implantationsvorrichtung nach Ansprüche 1 bis 16, **dadurch gekennzeichnet, daß** der Hohlkörper (27, 36, 63) des Katheters (20,35,90) aus einem elastischen Material, etwa Kunststoff oder einem elastischen Metall, wie Nitinol, besteht, und sein bei bestimmungsgemäßem Gebrauch vorderer Abschnitt (26) unter Wahrung eines im wesentlichen gleichbleibenden Innenquerschnitts umbiegbar ist.

18. Implantationsvorrichtung nach einem der Ansprüche 1 bis 17, **gekennzeichnet durch** einen im Hohlkörper (27, 36, 63) des Katheters (20,35,90) axialbeweglich aufnehmbaren Pusher (28, 65, 134), mittels dessen das Befestigungsmitte] (4, 8, 11-16,47,62) aus dem Katheter (20, 35, 90) hinausschiebbar ist.

19. Implantationsvorrichtung nach Anspruch 18, **dadurch gekennzeichnet, daß** das Befestigungsmittel (62) spiral- oder schraubenförmig ausgebildet ist und an seinem im implantierten Zustand von der Gefäßwand entgegengesetzten Ende eine Kupplung (66) zum drehfesten, aber lösbaren Verbinden mit einem entsprechenden Kupplungsabschnitt (68) des Pushers (65) aufweist.

20. Implantationsvorrichtung nach einem der Ansprüche 1 bis 19, **dadurch gekennzeichnet, daß** der Hohlkörper (27, 36, 63) des Katheters (20, 35, 90) wenigstens über einen Teil seiner Längserstreckung in einer Hülse (38)aus vorzugsweise elastischem Material aufgenommen ist.

21. Implantationsvorrichtung nach einem der Ansprüche 1 bis 20, **dadurch gekennzeichnet, daß** der Vorderabschnitt des Hohlkörpers (27, 36, 63) mit einem sich im wesentlichen über die gesamte Längserstreckung des Katheters (20, 35, 90) axialabeweglich ersteckenden Filament (40, 41) verbunden ist, mittels dessen der Vorderabschnitt des Hohlkörpers (27, 36, 63) umbiegbar ist.

22. Implantationsvorrichtung nach Anspruch 21, **dadurch gekennzeichnet, daß** das Filament (41) im Innern des Hohlkörpers (27, 36, 63), vorzugsweise in einem gesonderten Lumen (43), verläuft und das Umbiegen des Vorderabschnitts des Hohlkörpers (27, 36, 63) nach der Art eines Bowdenzugs erfolgt.

23. Imolantationsvorrichtung nach einem der Ansprüche 1 bis 22 **dadurch gekennzeichnet, daß** im bestimmungsgemäßen Beugungsbereich des Vorderabschnitts des Hohlkörpers (27,36,63) eine Armierung (42), vorzugsweise aus Metall, angeordnet ist.

24. Implantationsvorrichtung nach einem der Ansprüche 1 bis 23, **dadurch gekennzeichnet, daß** das Befestigungsmittel (4, 8, 11-16, 47, 62) zum Einbringen desselben in die Wandung des Hohlorgans (1) mit Federmitteln (100) in Wirkverbindung steht die bei der Einführung des Katheters (20, 35, 90) vorgespannt, jedoch nach lagerichtiger Plazierung des Katheters ( 20, 35, 90) im Hohlorgan (1) zur Beaufschlagung der Befestigungsmittel (4, 8, 11-16 47, 62) freigebbar sind.

25. lmplantationsvorrichtung nach einem der Ansprüche 1 bis 24, **dadurch gekennzeichnet, daß** mit den Befestigungsmittel (4, 8, 11-16, 47, 62) zum Einbringen desselben in die Organwandung ein elektromagnetischer Antrieb wirkverbunden ist.

26. Implantationsvorrichtung nach einem der Ansprüche 1 bis 23, **dadurch gekennzeichnet, daß** das Lume. des Hohlkörpers (27,36,63) mit einer Utraschallanordnung in Wirkverbindung steht, nittels der das Einbringen des Befestigungsmittels (4, 8, 11-16, 47, 62) in die Wandung des Hohlorgans (1) bewirkt oder unterstützt wird.

27. Implantationsvorrichtung nach einem der Ansprüche 1 bis 26, **dadurch gekennzeichnet, daß** im Hohlkörper (27, 36, 63) des Katheters ( 20, 35, 90) mehrere gleichzeitig implantierbare Befestigungsmittel (4, 8, 11-16, 47, 62) aufnehmbar sind.

28. Implantationsvorrichtung nach Anspruch 27, **dadurch gekennzeichnet, daß** der Vorderabschnitt des Hohlkörpers (27, 36, 63) mehrere während des Einführens des Katheters (20.35,90) paralle zueinander angeordnete, jedoch nach lagerichtiger Plazierung des Katheters (20, 35, 90) in verschiedene Richtungen umbiegbare Aufnahmeabschnitte (93,93',93") aufweist, die jeweils zur axialbeweglichen Aufnahme und Implantantation eines Befestigungsmittels (4, 8, 11-16, 47, 62) geeignet sind.

29. Implantationsvorrichtung nach einem der Ansprüche 1 bis 28, **dadurch gekennzeichnet, daß** im Hohlkörper (27, 36, 63) des Katheters (20, 35, 90) mehrere Befestigungsmittel (4, 8, 11-16, 47, 62) zur sequentiellen Implantation hintereinander axialbeweglich angeordnet sind.

30. Implantationsvorrichtung nach Anspruch 1 bis 29, **dadurch gekennzeichnet, daß** am Katheter (20, 35, 90) ein Magazin (113) mit Befestigungsmitteln (4, 8, 11-16, 47, 62) angeordnet ist, und mittels einer Ladevorrichtung (125) nach jeder Implantation eines Befestigungsmittels (4, 8, 11-16,47, 62) ein nachfolgendes Befestigungsmittel anstelle des implantierten Befestigungsmittels im Hohlkörper (27, 36, 63) plaziert wird.

31. Implantationsvorrichtung nach einem der Ansprüche 1 bis 30, **dadurch gekennzeichnet, daß** in einer Aufnahme (137) des Hohlkörpers (27, 36, 63) und/oder der Hülse (38) des Katheters (20, 35, 90) wenigstens ein elastisch streckbares Befestigungsmittel (4, 8, 11-16, 47, 62) axialbeweglich angeordnet ist, das nach erfolgter Implantation des jeweils vorhergehenden Befestigungsmittels (4, 8, 11-16,47,62) mit Hilfe eines in der Aufnahme (137) axialbeweglich aufgenommenen Ladepushers (138) in die zur Implantation geeignete Ausgangsposition des vorhergehenden Befestigungsmittels (4, 8, 11-16,47, 62) bringbar ist.

32. Implantationsvorrichtung nach einem der Ansprüche 1 bis 31, **dadurch gekennzeichnet, daß** der Hohlkörper (27, 36, 63) des Katheters (20, 35, 90) mit einem Zuführschlauch (133) verbunden ist, mittels dessen zur Implantation vorgesehene Befestigungsmittel (4, 8, 11-16. 47, 62) in den Hohlkörper (27, 36, 63), etwa mit Hilfe eines Zuführpushers (134), plazierbar sind.

33. Implantationsvorrichtung nach einem der Ansprüche 1 bis 33, **dadurch gekennzeichnet, daß** am Katheter (20,35,90) eine Spreizhülse (112) aus durch Längsschlitze voneinander getrennten Stegen (115,115') mit jeweils nach außen gerichteterVorkrümmung angeordnet ist, und mit dem ein am distalen Ende des Katheters herausragendes Zugglied (120) zum Aufspreizen der Stege (115,115) infolge Zugkraft fest verbunden ist.

34. Implantationsvorrichtung nach einem der Ansprüche 1 bis 33, **dadurch gekennzeichnet, daß** der Katheter (20, 35, 90) zumindest teilweise im Innenraum eines Ballonkatheters (141) aufgenommen ist und daß der Vorderabschnitt des Hohlkörpers (27, 36, 63, 89) des Katheters (20, 35, 90) im bestimmungsgemäßen Aufweitungszustand des Ballonkatheters (141) zur Gefäßwand hin umbiegbar ist.

35. Implantationsvorrichtung nach einem der Ansprüche 1 bis 34, **dadurch gekennzeichnet, daß** der Hohlkörper (27, 36, 63, 89) des Katheters (20, 35, 90) an seinem vorderen Ende angespitzt ist und im lmplantationszustand der lmplantationsvorrichtung (25, 45, 60, 86, 99, 102, 110, 130, 136, 140) mit der Wandung des Hohlorgans (1) lösbar verbunden ist.

36. lmplantationsvorrichtung nach einem der Ansprüche 1 bis 35, **dadurch gekennzeichnet, daß** als Mittel zum Aufnehmen und Positionieren der Schutzüberdeckung (72) mehrere, axialbeweglich in entsprechenden Aufnahmen des Hohlkörpers (27,36,63,89) des Katheters (20, 35, 90) aufgenommene und unabhängig voneinander betätigbare Positionierdrähte (132) vorgesehen sind.

37. Implantationsvorrichtung nach einem der Ansprüche 1 bis 36, **dadurch gekennzeichnet, daß** ein zumindest abschnittsweise als Hohlkörper ausgebildetes Befestigungsmittel (105,106) auf einer aus einem Filament bestehenden, zumindest abschnittsweise koaxial im Katheter verlaufenden Sonde (103) aufgenommen ist und der Katheter (20, 35, 90) als Pusher des als Hohlkörper ausgebildeten Befestigungsmittels (105, 106) einsetzbar ist.

38. Implantationsvorrichtung nach einem der Ansprüche 1 bis 37, **dadurch gekennzeichnet, daß** zur laufenden Überwachung des Implantationsvorgangs am Katheter (20, 35, 90) eine mit einem Bildgeber wirkverbundene Ultraschalldiagnoseeinrichtung (121) angeordnet ist.

## Claims

1. An implantation device for the treatment of damaged or diseased tissue within the region of the inner walls of hollow organs, in particular an implantation device for treating a dissection in body vessels, with a catheter (20, 35, 90) and at least one securing means (4, 8, 11-16, 47, 62) connected with this catheter (20, 35, 90), wherein the catheter (20, 35, 90) has an elongate hollow body which is open at least at its end at the front when used as intended and in which each securing means (4, 8, 11-16, 47, 62) is axially movably received when the implantation device (25, 45, 60, 85, 99, 102, 110, 130, 136, 140) is inserted into the body vessel (1), **characterised in that** the hollow body (27, 36, 63) of the catheter (20, 35, 90) is bent up at its front end and thereby each securing means (4, 8, 11-16, 47, 62) after positionally correct placement of the catheter (20, 35, 90) in the hollow organ (1) is implantable by at least one implantation section (9, 96) in the wall of the hollow organ (1) in targeted manner and is subsequently detachable from the catheter (20, 35, 90).

2. Implantation device according to claim 1, **characterised in that** the catheter (20, 35, 90) has means (132) for receiving and positioning a protective cover (72) for the affected tissue, said means being detachable from the catheter (20, 35, 90) after placement of the catheter (20, 35, 90) in the correct position in the hollow organ (1), and securable to the inner wall of the hollow organ (1) by means of at least one securing means (4, 8, 11-16, 47, 62).

3. Implantation device according to claim 2, **characterised in that** the protective covering (72) is a piece of textile fabric or a preferably porous plastic foil adapted to the area of the damaged tissue.

4. Implantation device according to claim 3, **characterised in that** in the textile structure is a hose-like graft (80, 87).

5. Implantation device according to any one of claims 1 to 4, **characterised in that** a nail (4, 8, 11) is provided as securing means, the shank (9) of the nail forming the implantation section of the securing means (4, 8, 11-16, 47, 62).

6. Implantation device according to any one of claims 1 to 4, **characterised in that** the implantation section (9, 95) of the securing means (4, 8, 11-16, 47, 62) is constructed in the form of a staple (13-16, 47) having at least two legs, which are arranged in U-shape or V-shape relative to each other.

7. Implantation device according to any one of claims 1 to 6, **characterised in that** the implantation section (9, 95) of the securing means (4, 8, 11-16, 47, 62) consists of at least two legs which are resiliently connected together at one end and which are arranged substantially in V-shape relative to each other in the intended position of implantation of the securing means (4, 8, 11-16, 47, 62).

8. Implantation device according to any one of claims 1 to 7, **characterised in that** the implantation section (9, 95) of the securing means (4, 8, 11-16, 47, 62) is provided with barbs (19, 19').

9. Implantation device according to any one of claims 1 to 8, **characterised in that** the securing means (4, 8, 11-16, 47, 62) consists of a biodegradable material.

10. Implantation device according to any one of claims 1 to 9, **characterised in that** the securing means (4, 8, 11-16, 47, 62) consists of an elastic material, for example "Nitinol".

11. Implantation device according to any one of claims 1 to 4, **characterised in that** a material which is liquid during the implantation, but curing within a predetermined period of time is used for the securing means (4, 8, 11-16, 47, 62).

12. Implantation device according to any one of claims 1 to 11, **characterised in that** the securing means (4, 8, 11-16, 47, 62) is provided with a diagnostic contrast medium or consists of a material providing contrast in a selected diagnostic method.

13. Implantation device according to any one of claims 1 to 12, **characterised in that** the securing means (4, 8, 11-16, 47, 62) is suitable for reception and controlled dispensing of a predetermined amount of medications.

14. Implantation device according to claim 13, **characterised in that** the securing means (4, 8, 11-16, 47, 62) is provided with a cavity for receiving a predetermined amount of medications.

15. Implantation device according to claim 14, **characterised in that** the securing means (4, 8, 11-16, 47, 62) is biodegradable and that the medication is released upon decomposition of the securing means.

16. Implantation device according to claim 13, **characterised in that** for receiving medications there is provided at least one medication pouch (77) which is connected with a head section (10) of the securing means (4, 8, 11-16, 47, 62) and which is provided with apertures (78) for dispensing the medication storable in the medication pouch (77) at a predetermined rate.

17. Implantation device according to claims 1 to 16, **characterised in that** the hollow body (27, 36, 63) of the catheter (20, 35, 90) consists of an elastic material such as, for example, plastic, or an elastic metal such as "Nitinol" and its section (26) at front when used as intended can be bent over whilst maintaining a substantially unchanged inner cross-section.

18. Implantation device according to any one of claims 1 to 17, **characterised by** a pusher (28, 65, 134) which is axially movably receivable in the hollow body (27, 36, 63) of the catheter (20, 35, 90) and by means of which the securing means (4, 8, 11-16, 47, 62) can be pushed out of the catheter (20, 35, 90).

19. Implantation device according to claim 18, **characterised in that** the securing means (62) is constructed to be spiral or helical and, at its end opposite the vessel wall in the implanted state, has a coupling (66) for rotationally fast, but detachable connection with a corresponding coupling section (68) of the pusher (65).

20. Implantation device according to any one of claims 1 to 19, **characterised in that** the hollow body (27, 36, 63) of the catheter (20, 35, 90) is received over at least part of its longitudinal extent in a sleeve (38) preferably of elastic material.

21. Implantation device according to any one of claims 1 to 20, **characterised in that** the front section of the hollow body (27, 36, 63) is connected with a filament (40, 41) which extends in axial movable manner substantially over the entire longitudinal extent of the catheter (20, 35, 90) and by means of which the front section of the hollow body (27, 36, 63) can be bent over.

22. Implantation device according to claim 21, **characterised in that** the filament (41) extends in the interior of the hollow body (27, 36, 63), preferably in a separate lumen (43), and the bending over of the front section of the hollow body (27, 36, 63) is effected in the manner of a Bowden pull.

23. Implantation device according to any one of claims 1 to 22, **characterised in that** a reinforcement (42), preferably of metal, is arranged within the intended range of deflection of the front section of the hollow body (27, 36, 63).

24. Implantation device according to any one of claims 1 to 23, **characterised in that** the securing means (4, 8, 11-16, 47; 62) is operatively connected with spring means (100) for insertion thereof into the wall of the hollow organ (1), the spring means being biassed on insertion of the catheter (20, 35, 90), but being releasable after positionally correct placement of the catheter (20, 35, 90) in the hollow organ (1) for acting on the securing means (4, 8, 11-16, 47, 62).

25. Implantation device according to any one of claims 1 to 24, **characterised in that** an electromagnetic drive is operatively connected with the securing means (4, 8, 11-16, 47, 62) for inserting the latter into the wall of the organ.

26. Implantation device according to any one of claims 1 to 23, **characterised in that** the lumen of the hollow body (27, 36, 63) is operatively connected with an ultrasound arrangement, by means of which the insertion of the securing means (4, 8, 11-16, 47, 62) into the wall of the hollow organ (1) is effected or assisted.

27. Implantation device according to any one of claims 1 to 26, **characterised in that** several simultaneously implantable securing means (4, 8, 11-16, 47, 62) are receivable in the hollow body (27, 36, 63) of the catheter (20, 35, 90).

28. Implantation device according to claim 27, **characterised in that** the front section of the hollow body (27, 36, 63) has a plurality of receiving sections (93, 93', 93") which are arranged parallel with each other during insertion of the catheter (20, 35, 90), but can be bent over in different directions after positionally correct placement of the catheter (20, 35, 90), and which are each suitable for axially movable reception and implanting of a securing means (4, 8, 11-16, 47, 62).

29. Implantation device according to any one of claims 1 to 28, **characterised in that** several securing means (4, 8, 11-16, 47, 62) are arranged with axial mobility in succession in the hollow body (27, 36, 63) of the catheter (20, 35, 90) for sequential implantation.

30. Implantation device according to claims 1 to 29, **characterised in that** a magazine (113) with securing means (4, 8, 11-16, 47, 62) is arranged at the catheter (20, 35, 90) and following each implantation of a securing means (4, 8, 11-16, 47, 62) a succeeding securing means is placed in the hollow body (27, 36, 63) by means of a loading device (125) in place of the implanted securing means.

31. Implantation device according to any one of claims 1 to 30, **characterised in that** at least one elastically stretchable securing means (4, 8, 11-16, 47, 62) is arranged in a receptacle (137) of the hollow body (27, 36, 63) and/or of the sleeve (38) of the catheter (20, 35, 90) to be axially movable and, after implantation of the respective preceding securing means (4, 8, 11-16, 47, 62), can be brought into the starting position, which is suitable for implantation, of the preceding securing means (4, 8, 11-16, 47, 62) with the help of a loading pusher (138) axially movably received in the receptacle (137).

32. Implantation device according to any one of claims 1 to 31, **characterised in that** the hollow body (27, 36, 63) of the catheter (20, 35, 90) is connected with a feed hose (133) by means of which the securing means (4, 8, 11-16, 47, 62) intended for implantation in the hollow body (27, 36, 63) can be placed, for example with the help of a feed pusher (134).

33. Implantation device according to any one of claims 1 to 32, **characterised in that** a spreading sleeve (112) consisting of bridges (115, 115') separated from each other by longitudinal slits and each with an outwardly directed preliminary curvature is arranged at the catheter (20, 35, 90), and a pulling element (120) projecting from the distal end of the catheter is solidly connected with the catheter for spreading the bridges (115, 155') by tensile force.

34. Implantation device according to any one of claims 1 to 33, **characterised in that** the catheter (20, 35, 90) is at least partly received in the interior of a balloon catheter (141) and that the front section of the hollow body (27, 36, 63, 89) of the catheter (20, 35, 90) can be bent over towards the vessel wall when the balloon catheter (141) is in its intended inflated state.

35. Implantation device according to any one of claims 1 to 34, **characterised in that** the hollow body (27, 36, 63, 89) of the catheter (20, 35, 90) is pointed at its front end and detachably connected with the wall of the hollow organ (1) when the implantation device (25, 45, 60, 85, 99, 102, 110, 130, 136, 140) is in the implanted state.

36. Implantation device according to any one of claims 1 to 35, **characterised in that** as means for receiving and positioning the protective covering (72) there is provided a plurality of positioning wires (132) which are received in corresponding receptacles of the hollow body (27, 36, 63, 89) of the catheter (20, 35, 90) to be axially movable and are actuable independently of each other.

37. Implantation device according to any one of claims 1 to 36, **characterised in that** a securing means (105, 106) constructed at least in sections as a hollow body is received on a probe (103) consisting of a filament and extending at least by sections coaxially in the catheter and the catheter (20, 35, 90) is usable as a pusher of the securing means (105, 106) constructed as a hollow body.

38. Implantation device according to any one of claims 1 to 37, **characterised in that** a diagnostic ultrasound system (121) operatively connected with an image emitter is arranged at the catheter (20, 35, 90) for continuously monitoring the implantation process.

## Revendications

1. Dispositif d'implantation pour le traitement d'un tissu blessé ou atteint au voisinage de la paroi intérieure d'organes creux, notamment dispositif d'implantation pour le traitement d'une dissection dans des vaisseaux corporels,
avec un cathéter (20, 35, 90) et au moins un moyen de fixation (4, 8, 11-16, 47, 62) relié à ce cathéter (20, 35, 90),
où le cathéter (20, 35, 90) présente un corps creux oblong, ouvert au moins à son extrémité avant lors d'une utilisation prescrite, dans lequel chaque moyen de fixation (4, 8, 11-16, 47, 62) lors de l'insertion du dispositif d'implantation (25, 45, 60, 85, 99, 102, 110, 130, 136, 140) est reçu d'une manière déplaçable axialement dans le vaisseau corporel (1),
**caractérisé en ce que** le corps creux (27, 36, 63) du cathéter (20, 35, 90) est recourbé à son extrémité avant et que de ce fait, chaque moyen de fixation (4, 8, 11-16, 47, 62), après une mise en place dans la bonne position du cathéter (20, 35, 90) dans l'organe creux (1), peut être implanté respectivement d'une manière ciblée par au moins un tronçon d'implantation (9, 96) dans la paroi de l'organe creux (1) et peut être détaché ensuite du cathéter (20, 35, 90).

2. Dispositif d'implantation selon la revendication 1, **caractérisé en ce que** le cathéter (20, 35, 90) présente des moyens (132) pour la réception et le positionnement d'un recouvrement de protection (72) pour le tissu concerné qui, après la mise en place dans la bonne position du cathéter (20, 35, 90) dans l'organe creux (1) peut être détaché du cathéter (20, 35, 90) et peut être fixé avec au moins un moyen de fixation (4, 8, 11-16, 47, 62) à la paroi intérieure de l'organe creux (1).

3. Dispositif d'implantation selon la revendication 2, **caractérisé en ce que** dans le cas du recouvrement de protection (72), il s'agit d'une partie de tissu textile adaptée à la zone de tissu endommagée ou d'une feuille de matière synthétique, de préférence poreuse.

4. Dispositif d'implantation selon la revendication 3, **caractérisé en ce qu'**il s'agit dans le cas de la structure textile d'une greffe tubulaire (80, 87).

5. Dispositif d'implantation selon l'une des revendications 1 à 4, **caractérisé en ce qu'**il est prévu comme moyen de fixation un clou(4, 8, 11) dont la tige (9) forme le tronçon d'implantation du moyen de fixation (4, 8, 11-16, 47, 62).

6. Dispositif d'implantation selon l'une des revendications 1 à 4, **caractérisé en ce que** le tronçon d'implantation (9, 95) du moyen de fixation (4, 8, 11-16, 47, 62) est réalisé comme un crampon (13-16, 47) présentant au moins deux branches, les branches étant disposées respectivement en forme de U ou de V l'une relativement à l'autre.

7. Dispositif d'implantation selon l'une des revendications 1 à 6, **caractérisé en ce que** le tronçon d'implantation (9, 95) du moyen de fixation (4, 8, 11-16, 47, 62) est constitué d'au moins deux branches élastiques reliées à respectivement une extrémité l'une à l'autre qui sont disposées dans la position d'implantation prescrite du moyen de fixation (4, 8, 11-16, 47, 62) respectivement sensiblement en forme de V l'une vers l'autre;

8. Dispositif d'implantation selon l'une des revendications 1 à 7, **caractérisé en ce que** le tronçon d'implantation (9, 95) du moyen de fixation (4, 8, 11-16, 47, 62) est pourvu de barbes (19, 19').

9. Dispositif d'implantation selon l'une des revendications 1 à 8, **caractérisé en ce que** le moyen de fixation (4, 8, 11-16, 47, 62) est constitué d'un matériau biologiquement dégradable.

10. Dispositif d'implantation selon l'une des revendications 1 à 9, **caractérisé en ce que** le moyen de fixation (4, 8, 11-16, 47, 62) est constitué d'un matériau élastique, par exemple de nitinol.

11. Dispositif d'implantation selon l'une des revendications 1 à 4, **caractérisé en ce qu'**on utilise pour le moyen de fixation (4, 8, 11-16, 47, 62) un matériau liquide lors de l'implantation, qui durcit cependant à l'intérieur d'une durée prédéterminée.

12. Dispositif d'implantation selon l'une des revendications 1 à 11, **caractérisé en ce que** le moyen de fixation (4, 8, 11-16, 47, 62) est pourvu d'un produit de contraste de diagnostic ou est constitué d'un matériau générateur de contraste dans un procédé de diagnostic sélectionné.

13. Dispositif d'implantation selon l'une des revendications 1 à 12, **caractérisé en ce que** le moyen de fixation (4, 8, 11-16, 47, 62) convient pour la réception et l'émission contrôlées d'une quantité prédéterminée de médicaments.

14. Dispositif d'implantation selon la revendication 13, **caractérisé en ce que** le moyen de fixation (4, 8, 11-16, 47, 62) est pourvu d'un espace creux pour la réception d'une quantité prédéterminée de médicaments.

15. Dispositif d'implantation selon la revendication 14, **caractérisé en ce que** le moyen de fixation (4, 8, 11-16, 47, 62) est dégradable biologiquement et que les médicaments sont libérés lors de la décomposition du moyen de fixation.

16. Dispositif d'implantation selon la revendication 13, **caractérisé en ce qu'**il est prévu pour la réception de médicaments au moins un sachet de médicaments (77) relié à un tronçon de tête (10) du moyen de fixation (4, 8, 11-16, 47, 62) qui présente des ouvertures (78) pour la distribution du médicament pouvant être stocké dans le sachet de médicaments (77) en une quantité prédéterminée.

17. Dispositif d'implantation selon les revendications 1 à 16, **caractérisé en ce que** le corps creux (27, 36, 63) du cathéter (20, 35, 90) est constitué d'un matériau élastique, par exemple de matière synthétique ou d'un métal élastique, comme le nitinol et que son tronçon (26) avant lors de l'utilisation prescrite, en conservant une section transversale intérieure sensiblement constante, peut être recourbé.

18. Dispositif d'implantation selon l'une des revendications 1 à 17, **caractérisé par** un poussoir (28, 65, 134) pouvant être reçu d'une manière déplaçable axialement dans le corps creux (27, 36, 63) du cathéter (20, 35, 90) au moyen duquel le moyen de fixation (4, 8, 11-16, 47, 62) peut être poussé hors du cathéter (20, 35, 90).

19. Dispositif d'implantation selon la revendication 18, **caractérisé en ce que** le moyen de fixation (62) est réalisé en forme de spirale ou d'hélice et présente à son extrémité opposée à l'état implanté à la paroi du vaisseau un accouplement (66) pour la liaison immobile en rotation mais relâchable avec un tronçon d'accouplement correspondant (68) du poussoir (65).

20. Dispositif d'implantation selon l'une des revendications 1 à 19, **caractérisé en ce que** le corps creux (27, 36, 63) du cathéter (20, 35, 90) est reçu au moins sur une partie de son extension longitudinale dans un manchon (38), de préférence en matériau élastique.

21. Dispositif d'implantation selon l'une des revendications 1 à 20, **caractérisé en ce que** le tronçon avant du corps creux (27, 36, 63) est relié à un filament (40, 41) s'étendant d'une manière axialement mobile sensiblement sur toute l'extension longitudinale du cathéter (20, 35, 90), au moyen duquel le tronçon avant du corps creux (27, 36, 63) peut être recourbé.

22. Dispositif d'implantation selon la revendication 21, **caractérisé en ce que** le filament (41) s'étend dans l'intérieur du corps creux (27, 36, 63), de préférence dans une lumière séparée (43) et que le recourbement du tronçon avant du corps creux (27, 36, 63) a lieu à la manière d'un câble Bowden.

23. Dispositif d'implantation selon l'une des revendications 1 à 22, **caractérisé en ce qu'**il est disposé dans la zone de pliage prescrite du tronçon avant du corps creux (27, 36, 63) une armature (42), de préférence en métal.

24. Dispositif d'implantation selon l'une des revendications 1 à 23, **caractérisé en ce que** le moyen de fixation (4, 8, 11-16, 47, 62), pour introduire celui-ci dans la paroi de l'organe creux (1), est en liaison active avec des moyens à ressort (100) qui, lors de l'insertion du cathéter (20, 35, 90) sont précontraints mais qui, après une mise en place dans la bonne position du cathéter (20, 35, 90) peuvent cependant être libérés dans l'organe creux (1) pour solliciter les moyens de fixation (4, 8, 11-16, 47, 62).

25. Dispositif d'implantation selon l'une des revendications 1 à 24, **caractérisé en ce qu'**un dispositif d'entraînement électromagnétique est relié activement au moyen de fixation (4, 8, 11-16, 47, 62) pour introduire celui-ci dans la paroi de l'organe.

26. Dispositif d'implantation selon l'une des revendications 1 à 23, **caractérisé en ce que** la lumière du corps creux (27, 36, 63) est en liaison active avec un agencement à ultrasons au moyen duquel on provoque ou on contribue à l'introduction du moyen de fixation (4, 8, 11-16, 47, 62) dans la paroi de l'organe creux (1).

27. Dispositif d'implantation selon l'une des revendications 1 à 26, **caractérisé en ce que** plusieurs moyens de fixation implantés en même temps (4, 8, 11-16, 47, 62) peuvent être reçus dans le corps creux (27, 36, 63) du cathéter (20, 35, 90).

28. Dispositif d'implantation selon la revendication 27, **caractérisé en ce que** le tronçon avant du corps creux (27, 36, 63) présente plusieurs tronçons de réception (93, 93', 93") disposés parallèlement les uns aux autres pendant l'introduction du cathéter (20, 35, 90) mais pouvant cependant être recourbés dans différentes directions après la mise en place dans la bonne position du cathéter (20, 35, 90), qui conviennent respectivement pour la réception axialement mobile et l'implantation d'un moyen de fixation (4, 8, 11-16, 47, 62).

29. Dispositif d'implantation selon l'une des revendications 1 à 28, **caractérisé en ce que** sont disposés d'une manière axialement mobile, les uns à la suite des autres, dans le corps creux (27, 36, 63) du cathéter (20, 35, 90) plusieurs moyens de fixation (4, 8, 11-16, 47, 62) en vue de l'implantation séquentielle.

30. Dispositif d'implantation selon les revendications 1 à 29, **caractérisé en ce qu'**il est disposé au cathéter (20, 35, 90) un magasin (113) avec des moyens de fixation (4, 8, 11-16, 47, 62) et **en ce qu'**il est mis en place au moyen d'un dispositif de charge (125), après chaque implantation d'un moyen de fixation (4, 8, 11-16, 47, 62) un moyen de fixation suivant à la place du moyen de fixation implanté dans le corps creux (27, 36, 63).

31. Dispositif d'implantation selon l'une des revendications 1 à 30, **caractérisé en ce qu'**il est disposé d'une manière axialement mobile dans un logement (137) du corps creux (27, 36, 63) et/ou le manchon (38) du cathéter (20, 35, 90) au moins un moyen de fixation (4, 8, 11-16, 47, 62) étirable élastiquement qui, après l'implantation du moyen de fixation respectivement précédent (4, 8, 11-16, 47, 62) peut être amené à l'aide d'un poussoir de charge (138) reçu d'une manière axialement mobile dans le logement (137) dans la position de départ qui convient pour l'implantation du moyen de fixation précédent (4, 8, 11-16, 47, 62).

32. Dispositif d'implantation selon l'une des revendications 1 à 31, **caractérisé en ce que** le corps creux (27, 36, 63) du cathéter (20, 35, 90) est relié à un tuyau d'amenée (133) au moyen duquel des moyens de fixation (4, 8, 11-16, 47, 62) prévus pour l'implantation peuvent être placés dans le corps creux (27, 36, 63) par exemple à l'aide d'un poussoir d'amenée (134).

33. Dispositif d'implantation selon l'une des revendications 1 à 33, **caractérisé en ce qu'**il est disposé au cathéter (20, 35, 90) un manchon d'écartement (112) en barrettes (115, 115') séparées par des fentes longitudinales avec respectivement une courbure préalable orientée vers l'extérieur et auquel est relié solidement, par suite de la force de traction, un organe de traction (120) dépassant à l'extrémité distale du cathéter pour écarter les barrettes (115, 115');

34. Dispositif d'implantation selon l'une des revendications 1 à 33, **caractérisé en ce que** le cathéter (20, 35, 90) est reçu au moins partiellement dans l'espace intérieur d'un cathéter à ballon (141) et **en ce que** le tronçon avant du corps creux (27, 36, 63, 89) du cathéter (20, 35, 90) peut être courbé à l'état d'expansion prescrit du cathéter à ballon (141) vers la paroi du vaisseau.

35. Dispositif d'implantation selon l'une des revendications 1 à 34, **caractérisé en ce que** le corps creux (27, 36, 63, 89) du cathéter (20, 35, 90) est affûté à son extrémité avant et est relié amoviblement à l'état d'implantation du dispositif d'implantation (25, 45, 60, 86, 99, 102, 110, 130, 136, 140) à la paroi de l'organe creux (1).

36. Dispositif d'implantation selon l'une des revendications 1 à 35, **caractérisé en ce que** sont prévus comme moyens pour recevoir et positionner le recouvrement de protection (72) plusieurs fils de positionnement (132) reçus d'une manière axialement mobile dans des logements correspondants du corps creux (27, 36, 63, 89) du cathéter (20, 35, 90) et actionnables indépendamment les uns des autres.

37. Dispositif d'implantation selon l'une des revendications 1 à 36, **caractérisé en ce qu'**un moyen de fixation (105, 106) réalisé au moins par tronçons comme corps creux est reçu sur une sonde (103) constituée d'un filament, s'étendant au moins par tronçons coaxialement dans le cathéter et que le cathéter (20, 35, 90) est utilisable comme poussoir du moyen de fixation (105, 106) réalisé comme corps creux.

38. Dispositif d'implantation selon l'une des revendications 1 à 37, **caractérisé en ce qu'**il est disposé pour la surveillance continuelle de l'opération d'implantation au cathéter (20, 35, 90) une installation de diagnostic à ultrasons (121) fonctionnellement reliée à un écran.
